# EUROPEAN PATENT APPLICATION

(11) **EP 3 916 377 A1**
(43) Date of publication of application: **01.12.2021**
(21) Application number: 20744252.6
(22) Date of filing: 23.01.2020
(51) Int. Cl.: G01N 21/77, G01N 21/78, G01N 27/416, G01N 33/53, G01N 33/543

(54) **ANTIGEN MEASURING METHOD AND MEASURING APPARATUS**

(30) Priority: 25.01.2019 JP 2019010865
(71) Applicant: Provigate Inc., Tokyo 113-0033 (JP)
(72) Inventor: MIYAZAWA Yuuya, Tokyo 113-0033 (JP)
(74) Representative: Michalski Hüttermann & Partner Patentanwälte mbB
(86) International application number: PCT/JP2020/002431
(87) International publication number: WO 2020/153461

(57) **Abstract**

There is provided a method for measuring an antigen, comprising: providing a solution containing an antigen; providing a first antibody that specifically recognizes the antigen and is bound to a magnetic carrier; providing a second antibody that specifically recognizes the antigen and is modified with an oxidase; providing (a substrate liquid including) a substrate which reacts with the oxidase; allowing the first antibody to recognize the antigen;
allowing the second antibody to recognize the antigen; using a magnetic field to capture an antigen-antibody complex of the antigen recognized by the first antibody and the second antibody in the magnetic field; washing the antigen-antibody complex while it is captured in the magnetic field; reacting the substrate with the antigen-antibody complex to produce hydrogen peroxide; and measuring the hydrogen peroxide.

## Description

### TECHNICAL FIELD

The present disclosure relates to measurements of antigens.

### BACKGROUND ART

A number of methods for measuring various antigens have been developed. However, there are many large-sized configurations and many methods that require time and labor to complete the measurements.

For example, high performance liquid chromatography (HPLC) is difficult to miniaturize because a high-pressure pump is required to increase the speed of measurements. In addition, the apparatus is expensive, and periodic maintenance of the column is necessary. Other methods have been developed, such as enzymatic methods and immunological methods, which are simpler and can be measured in a short time than conventional HPLC methods. However, because of the need for high-precision optical apparatuses, the apparatuses required for the measurements are still expensive and tend to increase in size.

Therefore, there is a need for a simple and easily method and a miniaturized measuring apparatus of measuring various antigens.

### SUMMARY OF INVENTION

The present disclosure includes a measurement method and a measurement device of an antigen. A method for measuring an antigen according to an embodiment of the present disclosure may comprise providing a first antibody that specifically recognizes the antigen and is bound to a magnetic carrier, and preparing a second antibody that specifically recognizes the antigen and is modified with an oxidase. A magnetic field may be used to capture an antigen-antibody complex of the antigen recognized by the first antibody and the second antibody, in the magnetic field. The antigen-antibody complex may be washed in a captured state in the magnetic field. The antigen-antibody complex may be reacted with a substrate to produce hydrogen peroxide. The hydrogen peroxide may be measured.

### BRIEF DESCRIPTION OF DRAWINGS

FIG. 1A illustrates a diagram schematically showing a measurement of an antigen according to an embodiment.
FIG. 1B illustrates a diagram schematically showing a measurement of an antigen according to an embodiment.
FIG. 1C illustrates a diagram schematically showing a measurement of an antigen according to an embodiment.
FIG. ID illustrates a diagram schematically showing a measurement of an antigen according to an embodiment.
FIG. IE illustrates a diagram schematically showing a measurement of an antigen according to an embodiment.
FIG. 2 illustrates a flowchart showing an antigen measurement step according to an embodiment.
FIG. 3 illustrates a flowchart showing an antigen measurement step according to an embodiment.
FIG. 24 illustrates a flowchart showing an antigen measurement step according to an embodiment.

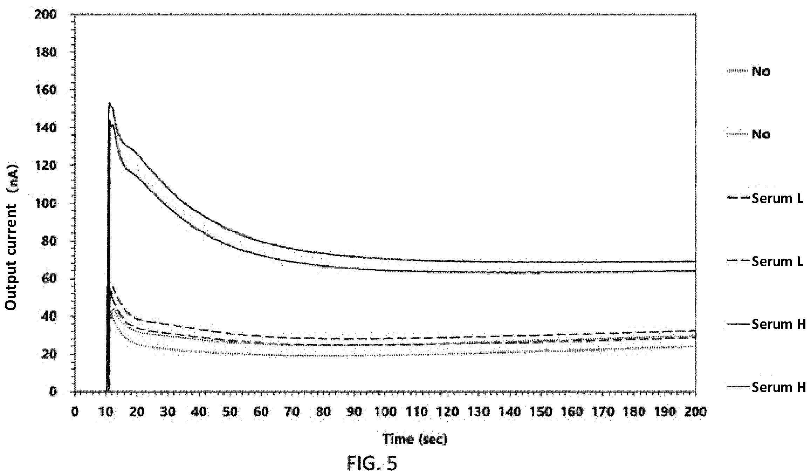

FIG. 5 shows a graph showing a temporal change of the measured output currents.

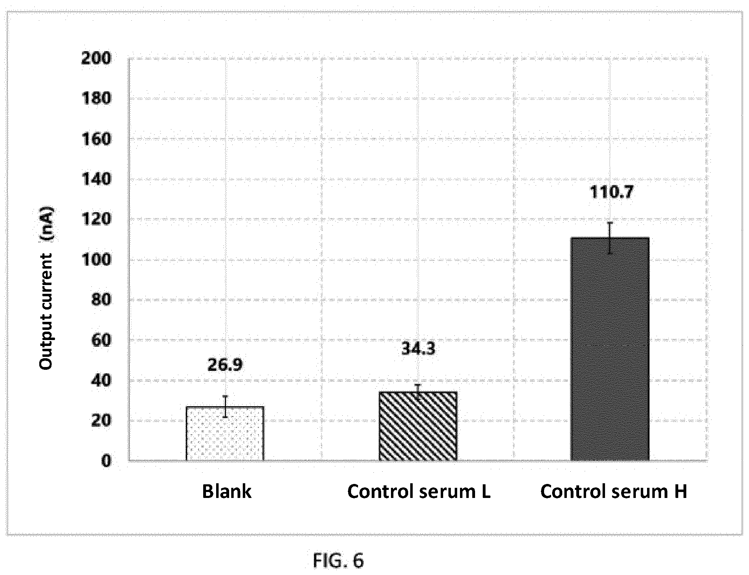

FIG. 6 shows a bar graph showing a comparison between samples of the current values shown in FIG. 5.

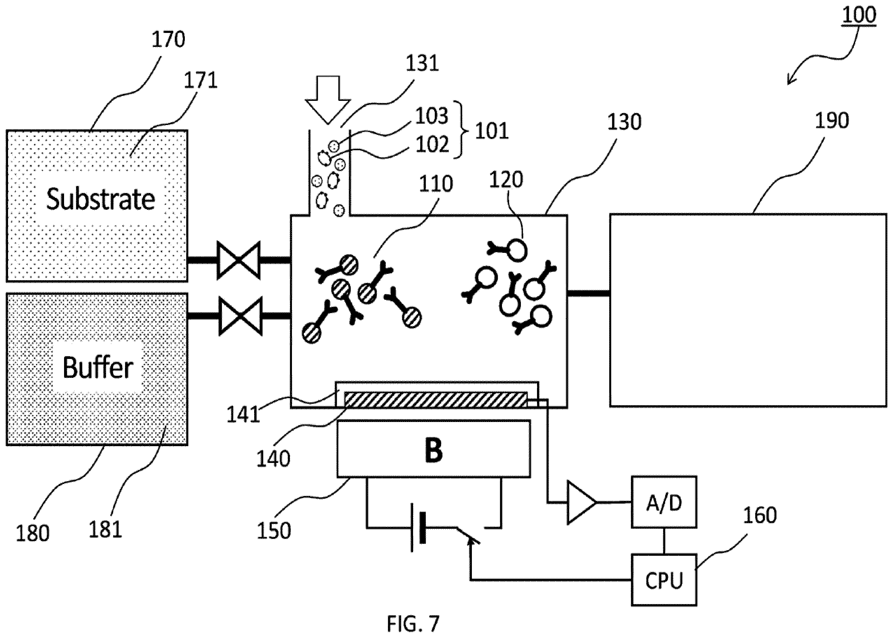

FIG. 7 illustrates a schematic diagram of an antigen measurement system according to an embodiment.

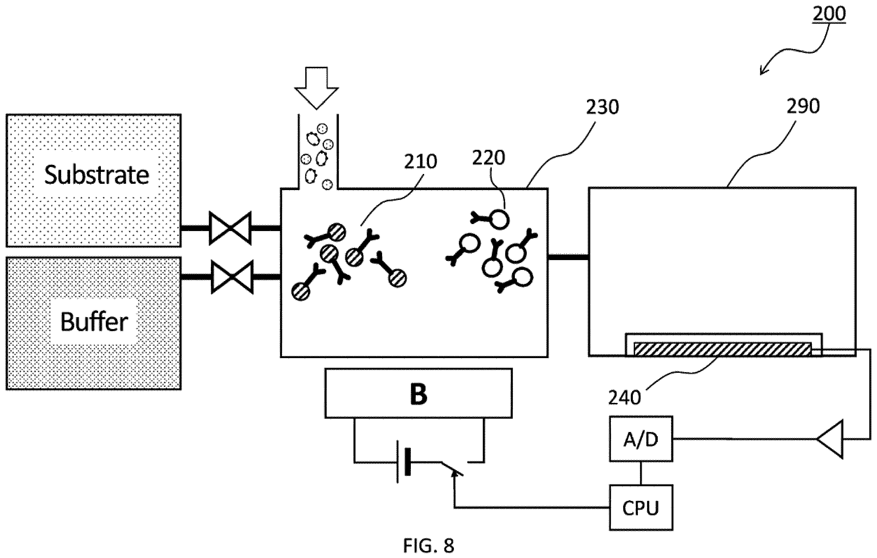

FIG. 8 illustrates a schematic diagram of an antigen measurement system according to an embodiment.

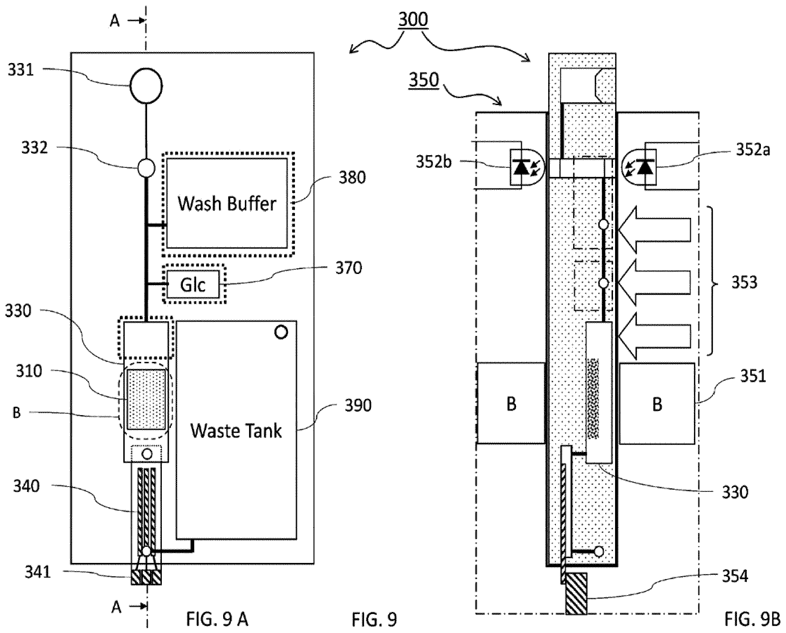

FIG. 9 illustrates a schematic diagram of an antigen measurement system according to an embodiment.

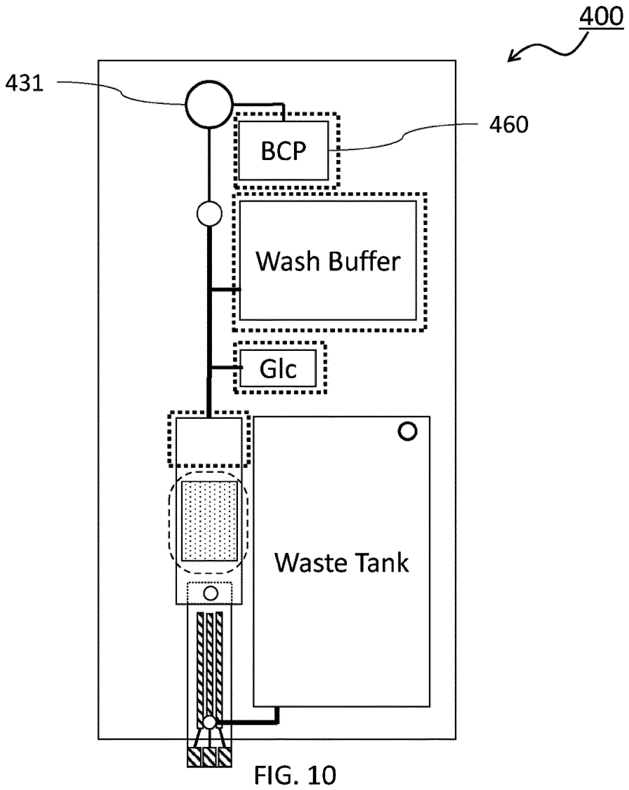

FIG. 10 illustrates a schematic diagram of an antigen measurement system according to an embodiment.

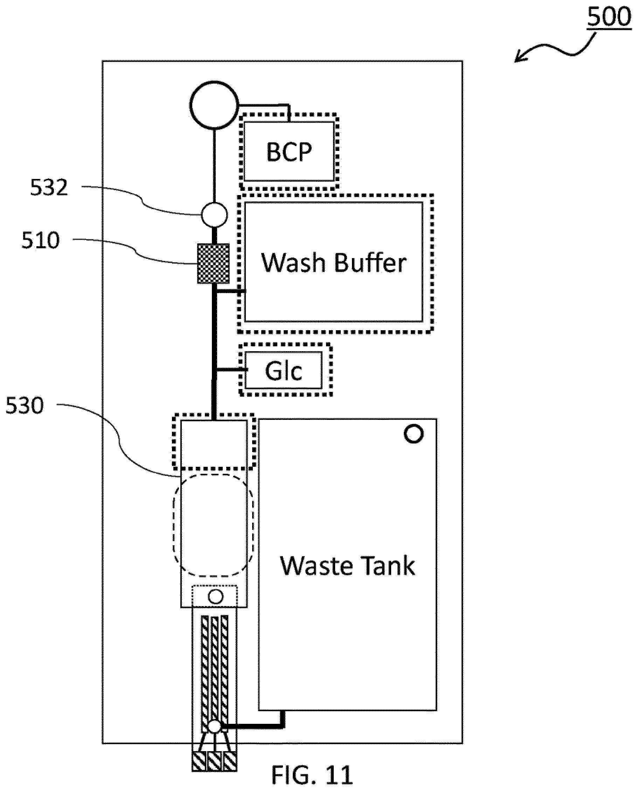

FIG. 11 illustrates a schematic diagram of an antigen measurement system according to an embodiment.

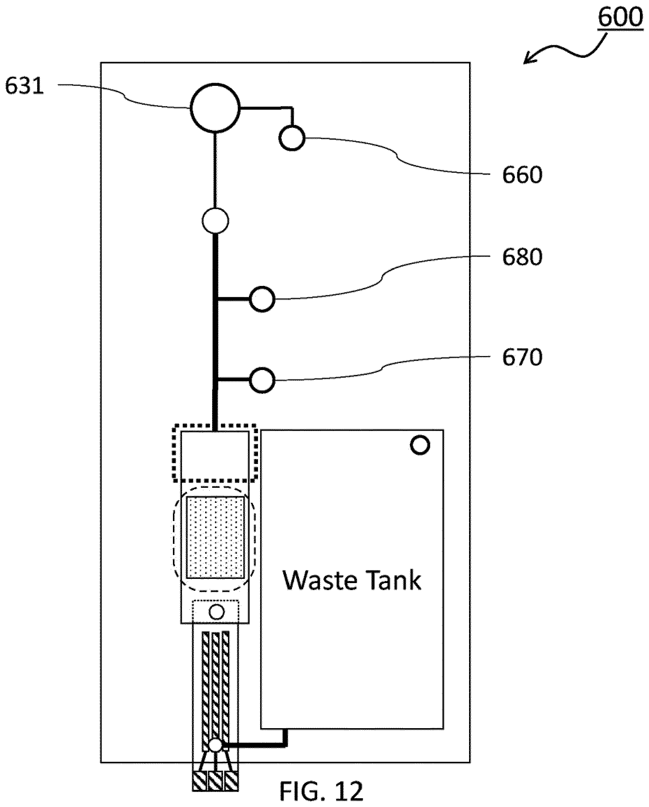

FIG. 12 illustrates a schematic diagram of an antigen measurement system according to an embodiment.

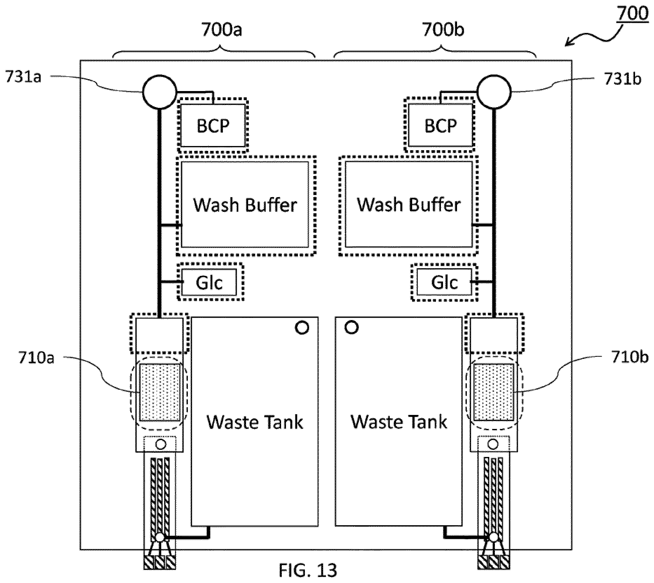

FIG. 13 illustrates a schematic diagram of an antigen measurement system according to an embodiment.

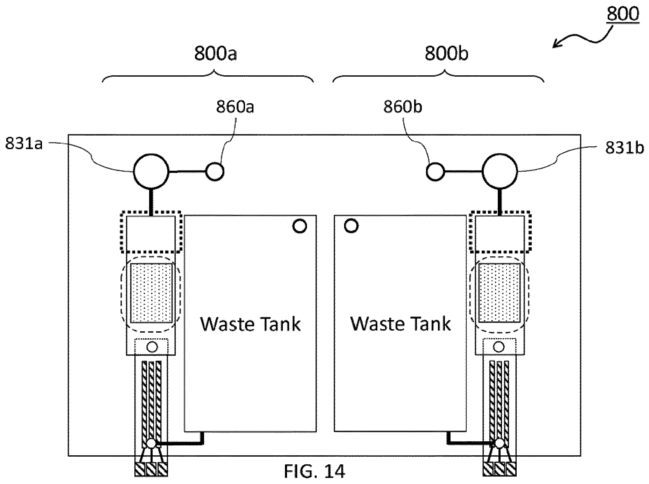

FIG. 14 illustrates a schematic diagram of an antigen measurement system according to an embodiment.

### DESCRIPTION OF EMBODIMENTS

### <Measurement Method>

A method for measuring an antigen according to an embodiment of the present disclosure may comprise providing a solution including an antigen (referred to as preparation and supply in the present disclosure);
providing a first antibody that specifically recognizes the antigen and is bound to a magnetic carrier;
providing a second antibody that specifically recognizes the antigen and is modified with an oxidase;
providing (a substrate liquid including) a substrate that reacts with the oxidase;
allowing the first antibody to recognize the antigen;
allowing the second antibody to recognize the antigen;
using a magnetic field to capture the antigen-antibody complex of the antigen recognized by the first antibody and the second antibody in the magnetic field;
washing the antigen-antibody complex while it is captured in the magnetic field;
reacting the substrate with the antigen-antibody complex to produce hydrogen peroxide; and
measuring the hydrogen peroxide.

The sample as measurement target may be a solution. The solution may be a body fluid, a solution derived from a body fluid, and may be a dilute fluid of a body fluid. The solution may be a solution that is not a body fluid (derived from a non-body fluid), and may be a mixture of a body fluid or a solution derived from a body fluid and a solution derived from a non-body fluid. The solution may be a solution used for a sample measurement and may be a solution used for a measurement for calibration. For example, the solution may be a standard solution or a calibration solution. The sample as measurement target may be a specimen.

The bodily fluid may be blood. The body fluid may be a solution derived from blood. The body fluid may be, for example, plasma, serum. The body fluid may be lymph fluid, tissue fluid such as interstitial fluid, intercellular fluid, interstitial fluid, and the like, and may be body cavity fluid, serosal fluid, pleural fluid, ascites fluid, capsular fluid, cerebrospinal fluid, joint fluid (synovial fluid), and aqueous humor of the eye (aqueous). The body fluid may be digestive fluid such as saliva, gastric juice, bile, pancreatic juice, intestinal fluid, and the like, and may be sweat, tears, nasal mucus, urine, semen, vaginal fluid, amniotic fluid, milk, and the like. The body fluid may be a body fluid of an animal and may be a body fluid of a human. "Body fluid" may be a solution. The solution may contain a physiological buffer such as phosphate buffered saline (PBS) or N-tris(hydroxymethyl)methyl-2-aminoethanesulfonic acid buffer (TES) containing a measurement target substance. The solution is not particularly limited as long as the measurement target substance is contained.

The solution may contain an antigen serving as a measurement target substance. For example, the solution may be tears and the measurement target substance may be glycoalbumin contained in tears. Alternatively, the measurement target substance may be albumin, glycoalbumin, hemoglobin, glycohemoglobin in blood or serum, albumin, glycoalbumin in interstitial fluid, albumin, glycoalbumin in tears, albumin, glycoalbumin in urine, or the like. The albumin may be oxidized albumin (HNA), reduced albumin (HMA).

In some embodiments, the antigen may be a protein. In some embodiments, the antigen may comprise a protein. In some embodiments, the protein may be a modified protein. The modification (modification) may be, for example, sugar chain addition, acetylation, phosphorylation, methylation, nitration, lipid addition, or the like. In some embodiments, the antigen may be a glycated protein.

In some embodiments, the antigen may be fructosamine. The fructosamine may be a glycated protein, may be a glycated peptide, and may be a glycated amino acid. The glycated protein may be glycated albumin and may be glycated hemoglobin. The glycated protein may be AGEs (Advanced Glycation End Products, terminal glycosylation products, late glycosylation products).

In some embodiments, the antigen may be an organism and may comprise an organism. For example, the antigen may be a bacterium, in which case the antibody may be, for example, an anti-E. coli antibody. For example, the antigen may be a virus. For example, the antibody may recognize a protein on the viral surface. When the antigen is a bacterium, an anti-pathogenic microbial antibody (a polychromatic antibody against E. coli, Listeria, Salmonella, Sera Care Inc.), an anti-infective pathogen antibody (a polychromatic antibody against Helicobacter, Staphylococcus, etc., Sera Care Inc), a pathogenic microbial monochromatic antibody (Multifunctional Protein Laboratories), or the like, for example may be used as an antibody. It does not have to recognize the bacterium itself. The Anti-E.coli LPS antibody (Abcam Inc.) may be used as an antibody using an antibody that recognizes an LPS antibody induced by E. coli as an antigen.

The antigen may be a hapten (incomplete antigen). The antigen may be a lipid, a nucleic acid, or a low molecule having a molecular weight of several hundred or less. The antigen may be a physiologically active substance.

A "magnetic carrier" is a carrier that includes a magnetic material that reacts to an external magnetic field. The material used for the magnetic carrier may be a diamagnetic material, a paramagnetic material, or a ferromagnetic material. The magnetic material may be a soft magnetic material or a hard magnetic material. The magnetic material may be iron oxide, chromium oxide, cobalt, ferrite, or the like.

The magnetic carrier may be a magnetic particle. The size of the magnetic carrier or particle may be greater than or equal to a value of 10 nm, 50 nm, 100 nm, 500 nm, 1 µm, or the like. The size of the magnetic carrier or particle may be smaller than or equal to a value of 100 µm, 75 µm, 50 µm, 40 µm, 30 µm, 25 µm, 20 µm, 10 µm, 1 µm, or the like.

The size of the magnetic particle may be appropriately adjusted. For example, a small magnetic carrier may be susceptible to forces due to factors other than magnetic fields, such as Brownian motion in solution. It is considered that when the force due to a factor other than the magnetic field increases, it becomes difficult to capture the antigen-antibody complex having the magnetic carrier by the magnetic field. For example, it is conceivable that a large magnetic carrier tends to easily capture an antigen-antibody complex having a magnetic carrier with a magnetic field, while reducing the reaction efficiency because the surface area per unit volume is small, that is, the number of antibodies per unit volume is reduced. In general, increasing the particle surface area per unit volume increases the upper limit of capturing a test target substance in a trace sample, and can be expected to improve the detection limit, output, and sensitivity. However, the above idea is merely an inference and does not necessarily mean physical correctness, and the size of the magnetic particles may be selected to be contrary to the above idea.

The magnetic carrier and the antibody may be bound via an organic molecule, and may be substantially directly bound. They may be bound via a carbon chain such as an alkane. In some embodiments, an organic molecular structure such as a group such as a functional group, a characteristic group, or a substituent or a sugar chain (hereinafter, simply referred to as a "group") on a surface of a magnetic carrier may be formed, or an antibody may be directly bound to a magnetic carrier on which a group such as a functional group, a sugar chain, and the like is exposed on the surface. Examples of the functional group include a carboxyl group, an amino group, a tosyl group, an aldehyde group, a maleimide group, a thiol group and the like. In some embodiments, a carbon chain may be bound to a functional group on a magnetic carrier surface and an antibody may be bound to the carbon chain. This exemplarily also allows the distance between the carrier and the antibody to be adjusted. In some embodiments, a carboxyl group, amino group, tosyl group, aldehyde group, maleimide group, thiol group on the magnetic surface may be bound to a carbon chain such as an alkane chain having a portion activated by N-hydroxysuccinimide or an alkane chain having a sugar chain, and an antibody may be linked to the other end. In some embodiments, a magnetic carrier and an antibody may be bound by physical adsorption or non-specific adsorption.

The first antibody and the second antibody may be substantially the same antibody and may comprise substantially the same antibody. The first antibody and the second antibody may include a different antibodies from each other, and each may comprise a substantially different antibody.

Both the first antibody and the second antibody may be monoclonal antibodies, one may be a polyclonal antibody and the other may be a monoclonal antibody, and both may be polyclonal antibodies. Each of the first antibody and the second antibody may comprise a single type of antibody, and may include or consist of a plurality of types of antibodies. Polyclonal antibodies may be more accessible and less expensive than monoclonal antibodies. Polyclonal antibodies may bind more to an antigen than monoclonal antibodies. More antibodies can be attached to the antigen, resulting in more measurement signals.

The antibody that recognizes a glycated protein may be monoclonal antibody and may be a polyclonal antibody. The monoclonal antibody that recognizes a glycated protein may be a monoclonal antibody capable of recognizing a glycation site of the protein. The antibody that recognizes a glycated protein may be a monoclonal antibody capable of recognizing a conformation generated by glycation of a protein. For example, higher order structures of a protein can deform the glycation sites by glycation. For example, a higher order structure of a protein can deform the tertiary structure in the vicinity of the glycation site by glycation. Higher order structures of a protein can deform the tertiary structure at a position different from the glycation site by glycation. Glycated proteins may have a different tertiary structure than non-glycated proteins. The glycosylation sites of glycated proteins recognized by monoclonal antibodies may recognize features unique to glycated proteins that differ from non-glycated proteins.

For example, Human Albumin Antibody Goat Polyclonal (BETHYL Laboratories Inc.) may be used as an anti-albumin polyclonal antibody. For example, anti-human albumin monoclonal FU-301 (Japan Biotest Laboratory, Inc.) may be used as a monoclonal antibody. For example, Monoclonal Antibody to Human Glycated Albumin Clone A717 (EXOCELL Corporation), Glycated Albumin monoclonal antibody (M02), clone 1A11 (Abnova Corporation), or the like may be used as an anti-GA monoclonal antibody. Anti-Phosphoserine, Rabbit-Poly (StressMArq Biosciensec Inc.) may be used as a polyclonal antibody for the phosphorylated peptide, and Phosphotyrosine (Clone, Biolegend Inc.) may be used as a monoclonal antibody. The above antibodies are merely examples and the antibodies of the present disclosure are not limited thereto. Other antibodies may be used. For the antigen-antibody reaction, for example, a method or a condition used in an antigen-antibody staining method such as Western blot may be employed.

"Oxidases" (oxidizing enzymes) are enzymes that use a substrate of molecular oxygen as an electron acceptor. Alternatively, oxidases are enzymes that catalyze redox reactions involving oxygen molecules as acceptors for hydrogen or electrons.

A "substrate" is a substance that catalyzes a chemical reaction by an enzyme. Alternatively, a substrate is a substance that binds to an enzymatic protein, and thereby undergoes a decrease in the activation energy of a particular chemical reaction upon binding to an enzyme and, as a result, is converted to a particular product at an amazing rate.

In some embodiments, the oxidase may include a glucose oxidase and a substrate may include a glucose. In some embodiments, the oxidase comprises an alcohol oxidase and the substrate may comprise a primary alcohol.

The magnetic field acting on the magnetic carrier may be generated using a magnet external to the measurement system or the reaction system, or may be generated using a magnet disposed inside the measurement system or the reaction system. The magnetic field may be generated by a permanent magnet, or may be generated by the supply of current. For example, a permanent magnet, or an electromagnet may be used to generate the magnetic field.

In order to allow an antibody to recognize an antigen, a liquid containing an antibody may be mixed with a solution containing an antigen. Various conditions and methods may be used for the antigen-antibody reaction. For example, the solution may be held at about 4°C overnight after mixing, and may be held at about 37°C for about 1 to 2 hours, and other temperatures and times may be employed. The pH may employ pH 6.5 to 8.4 or conditions close to in vivo, or other conditions. Various conditions may be employed for the ionic strength. For example, a commercially available PBS concentration (NaCl: 137 mM, KCl: 2.7 mM, Na₂HPO₄: 8.1 mM, KH₂PO₄: 1.47 mM) or the like may be used, and other ionic conditions may be employed.

In some embodiments, a magnetic field can be used to capture an antigen-antibody complex having a magnetic carrier in the magnetic field or to direct and capture it in a location outside the magnetic field. In some embodiments, another solution or liquid may be flowed where the antigen-antibody complex has been captured, in a state where the antigen-antibody complex is captured. Thus, for example, a molecule, an ion or other substance other than an antigen (measurement target substance) contained in a solution containing an antigen (a solution containing a measurement target substance), a so-called contaminant, can be poured out (also referred to as cleaning, washing, rinsing, flushing, or the like), or contaminants can be separated from the antigen or antigen-antibody complex. The sensitivity or accuracy of the measurement can be increased by measuring in a state where there are few contaminating parts.

The measurement or detection of hydrogen peroxide may be performed using a hydrogen peroxide sensor device. The measurement of hydrogen peroxide may be measuring the concentration of hydrogen peroxide.

The hydrogen peroxide sensor may be an electrode of an electrochemical type, and may be a hydrogen peroxide electrode. The hydrogen peroxide electrode may have a counter electrode, a reference electrode, and a working electrode. Hydrogen peroxide is decomposed at the hydrogen peroxide electrode, and the electrons emitted are detected as current. The reaction can be described as follows:

H₂O₂→2H + O₂ + 2e

This current is proportional to the concentration of hydrogen peroxide in the vicinity of the electrode. Under a steady-state, a quasi-steady-state or controlled or known conditions, the current is proportional or related to the concentration or amount of antigen in the original solution introduced. The current measurement is considered to be able to detect larger signals and can make more accurate measurements, and less susceptible to changes in ion concentration in a solution, compared to potential measurements. For example, in a voltage measurement, the ion concentration on the electrode surface may change the distance of the detection region (Debye length), resulting in a change in the S/N ratio. On the other hand, in the current measurement, it is considered that the S/N ratio does not easily change unless a redox current is generated on the electrode.

The measurement or detection of hydrogen peroxide may be performed using an optical method. The optical method may include a measurement of absorbance and emission. For example, by adding peroxidase and 4-aminoantipyrine and a color former, a color change of a quinone dye caused by oxidative condensation may be measured, for example, from a back surface of a transparent substrate. In some embodiments, the detector may include a luminescent reagent and a photodetector. For example, luminol may be used as a luminescent reagent. The luminol may be arranged in a powder form. Hydrogen peroxide may be reacted with luminol to measure the intensity of emission (wavelength 460 nm) by the luminol reaction. The reagents may further include potassium hexacyanoferrate, sodium hydroxide, and the like. The luminol reaction may be measured by an electrochemiluminescence method in which a gold electrode, a platinum electrode, or an indium tin oxide (ITO) transparent electrode is used and driven by an alternating current. Also, for detecting a fluorescent reaction, a combination of an oxalic acid ester and a fluorescent substance may be used, or lucigenin (acridinium) may be used. These optical systems can be relatively inexpensive and configured small.

For the measurement of hydrogen peroxide, methods other than the above may be used.

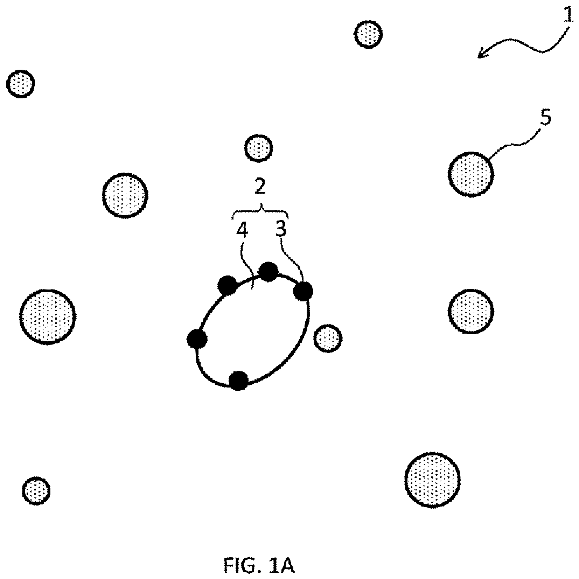

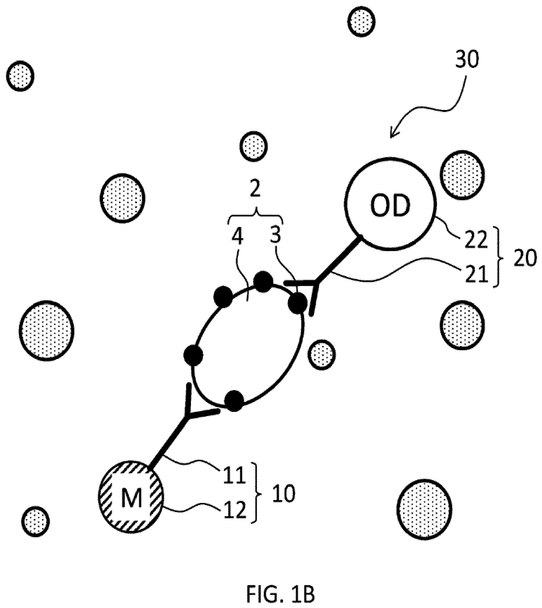

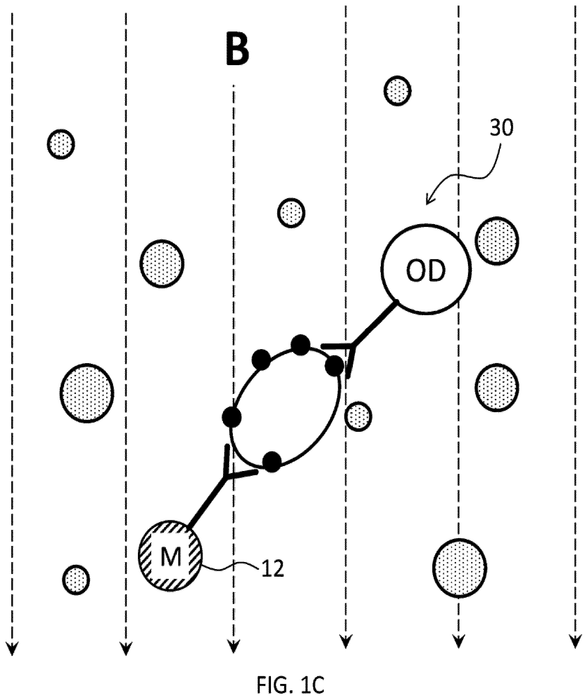

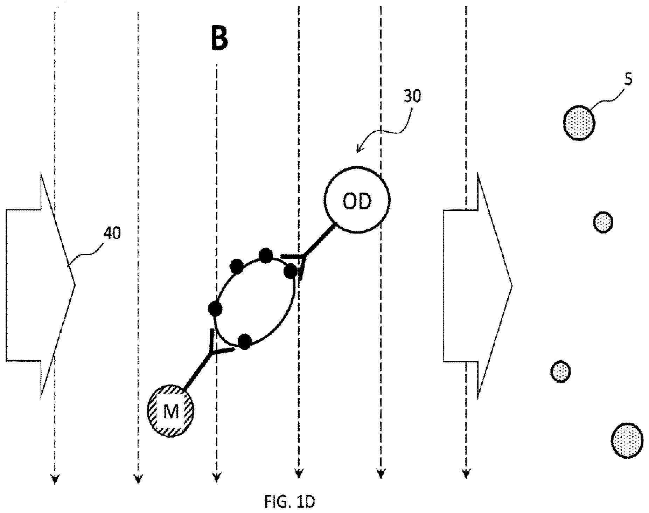

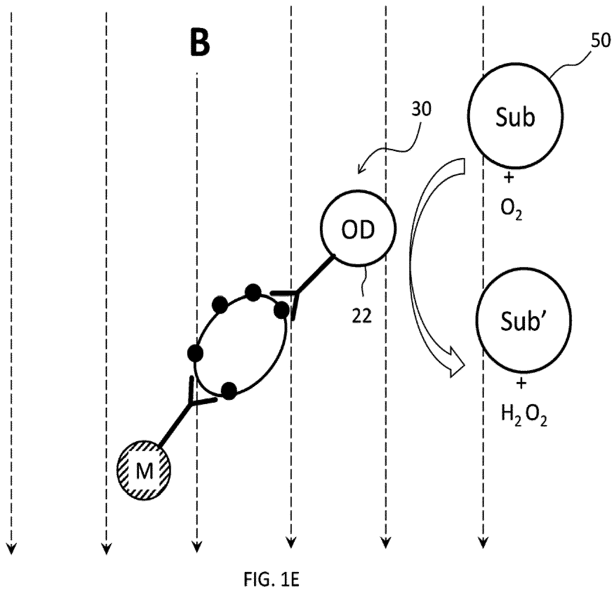

Hereinafter, a measurement procedure according to an embodiment will be described with reference to the drawings. FIG. 1 schematically shows a solution (antigen solution) 1 containing glycated protein 2 as an antigen. The glycated protein 2 is formed by binding a sugar 3 to a protein 4 body. The solution 1 also contains contaminants 5.

FIG. 1B schematically shows the specific recognition and binding of the two antibodies to the glycated protein 2. Two antibodies are bound to glycoprotein 2. The first antibody 10 is formed by binding an antibody 11 that recognizes a protein with a magnetic particle (M) 12. The other antibody 20 is formed by binding an antibody 21 that recognizes a protein with an oxidase (OD, Ox) 22. In FIG. 1B, the antibody 20 having the oxidase 22 is depicted as a monoclonal antibody that specifically recognizes a glycation site to which the sugar 3 in the glycation protein 2 is bound, and the antibody 10 having the magnetic particle 12 is depicted as a polyclonal antibody that specifically recognizes the body of the protein 4. In this way, in FIG. 1B, an antigen-antibody complex 30 is formed in which both the antibody 10 having the magnetic particle and the antibody 20 having the oxidase recognize the glycated protein 2.

However, the embodiments are not limited thereto. In other embodiments, the antibody 10 having the magnetic particle 12 may be a monoclonal antibody that specifically recognizes a glycation site to which the sugar 3 in the glycation protein 2 is bound, and the antibody 20 having the oxidase 22 may be a polyclonal antibody that specifically recognizes the body of protein 2. In some embodiments, both antibodies may be polyclonal antibodies. In some embodiments, both antibodies may be monoclonal antibodies.

In FIG. 1B, two antibodies are attached to one antigen (glycated protein 2), in other words the antibody 10 having one magnetic material 12, and the antibody 20 having one oxidase 22 are bound. In some embodiments, three or more antibodies may recognize an antigen to form an antigen-antibody complex. For example, two or more antibodies having a magnetic particle and/or two or more antibodies having a substrate may recognize an antigen to form an antigen-antibody complex.

In FIG. 1C, a magnetic field B is applied to the antigen-antibody complex 30. The magnetic field B makes the antigen-antibody complex 30, which is chemically integrated with the magnetic particle 12, less susceptible to movement of the fluid in the solution 1, thermal movement of surrounding molecules (e.g. Brownian motion), and the like. In other words, the antigen-antibody complex 30 having the magnetic particle 12 is captured in the magnetic field B.

In FIG. ID, a wash (rinse, flush) is schematically shown, which is performed in a state where the antigen-antibody complex 30 is captured in the magnetic field B. Here, wash solution or wash buffer 40 is flowed through. The antigen-antibody complex 30 is trapped in the magnetic field B and is substantially immobile relative to the magnetic field B. On the other hand, a substance other than the antigen-antibody complex 30, for example contaminant 5, does not react with the magnetic field B, so that it is separated out of the magnetic field B by being pushed in this manner into the wash solution 40 or together with the wash solution 40. Thus, in a certain space (here, a space to which the magnetic field B is applied), in this case, the glycated protein 2 as measurement target can be stayed in the form of the antigen-antibody complex 30, and the contaminants 5 which can affect the measurement from the same space can be discharged or excluded therefrom.

In FIG. IE, the generation of hydrogen peroxide from the antigen-antibody complex 30 is schematically shown. The antigen-antibody complex 30 is captured in the magnetic field B. Here a substrate 50 is introduced. The substrate 50 undergoes a catalytic reaction of the antigen-antibody complex 30 with the oxidase 22 to generate hydrogen peroxide (H₂O₂). Since this reaction is a catalytic reaction, if the substrate 50 is continued to be sufficiently fed, an amount of hydrogen peroxide proportional to the amount of oxidase 22 continues to be generated.

By measuring the generated hydrogen peroxide, the amount or concentration of oxidase may be determined from the amount or concentration of hydrogen peroxide. Since the amount of oxidase is proportional to the amount of the antigen-antibody complex 30, that is, the amount of the glycated protein 2 which is an antigen, the amount of the antigen contained in the antigen solution 1 can be determined.

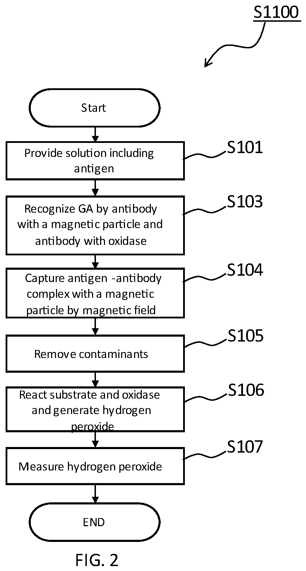

FIG. 2 shows a flow chart of a measuring process according to an embodiment (S1100). First, a solution containing an antigen is supplied (provided and prepared) (S1101). In some embodiments, the solution may be obtained by collecting bodily fluid. For example, the solution may be collected from tears, saliva, blood, or the like. For example, the solution may be plasma, serum, or the like. Next, an antibody having a magnetic particle and specifically recognizing a target antigen is introduced into the solution. At the same time, or before and after, an antibody having an oxidase and specifically recognizing an antigen is introduced. Thereby, an antigen-antibody complex in which the oxidase and the magnetic substance are bound is formed (S1103). A magnetic field is applied to the solution in which the antigen-antibody complex has been formed. The antigen-antibody complex that is bound to the magnetic particle is captured in this magnetic field (S1104). In the state where the antigen-antibody complex is captured in the magnetic field, a wash solution is flowed to separate or remove contaminants from the antigen-antibody complex (S1105). A solution containing a substrate (substrate liquid) is introduced into the remaining antigen-antibody complex. The substrate reacts with the oxidase of the antigen-antibody complex and hydrogen peroxide is generated (S1106). This hydrogen peroxide is measured (S1107). In some embodiments, the amount of hydrogen peroxide measured, temporal changes in that amount, or the like may be measured. In some embodiments, the concentration of antigen in the solution may be estimated from the measurement result of hydrogen peroxide, or the presence or absence of the antigen in the solution may be determined. The generated hydrogen peroxide may be measured by a hydrogen peroxide sensor (not shown).

In some embodiments, the antigen-antibody reaction may be performed in a reaction chamber in which a hydrogen peroxide sensor is disposed. In some embodiments, the formed antigen-antibody complex may be captured in the vicinity of the hydrogen peroxide sensor. In some embodiments, a magnetic field may be used to capture the formed antigen-antibody complex in the vicinity of the hydrogen peroxide sensor. In some embodiments, hydrogen peroxide may be generated by catalytic reaction of the oxidase of the antigen-antibody complex with the substrate while capturing the antigen-antibody complex in the vicinity of the hydrogen peroxide sensor. Thus, it is possible to increase the detection sensitivity of hydrogen peroxide by, for example, a hydrogen peroxide sensor.

In some embodiments, while the antigen-antibody complex is being captured in the magnetic field, the oxidase of the antigen-antibody complex and the substrate may be catalyzed to generate hydrogen peroxide, and the generated hydrogen peroxide may be separated from the antigen-antibody complex or separated out of the magnetic field. The separated hydrogen peroxide can be measured with a hydrogen peroxide sensor. By measuring hydrogen peroxide separated from the antigen-antibody complex, the measurement error due to the antigen-antibody complex can be reduced. For example, it is possible to reduce the influence on the measurement due to the presence of a substance other than hydrogen peroxide to be measured in the vicinity of the electrode during the measurement. For example, when the measurement is repeated, it affects the measurement such as an increase in measurement value in a carry-over of the previous antigen-antibody complex and the like. Therefore, the separation of hydrogen peroxide from the antigen-antibody complex eliminates the need for corrections with respect to the carry-over and can facilitate the measurement.

In some embodiments, the hydrogen peroxide sensor may be covered with a protective film. The protective film may, for example, eliminate or reduce the effect of contaminants on the hydrogen peroxide sensor. In some embodiments, the protective film may be formed on the hydrogen peroxide electrode. Thus, for example, non-specific adsorption of contaminants onto the electrode surface can be prevented or reduced. Alternatively, electric current that can result from redox reactions of the redox material with the electrode can be prevented or reduced.

The protective film may include a polymer film and may substantially consist of a polymer. For example, the protective film may be or may include a film having an alkane chain containing or having a hydrophilic group or a cellulose acetate film. The protective film may include a BSA film. The protective film may be a multilayer film. For example, a silane layer may be formed on the peroxide electrode, a film of an ion-exchangeable resin may be formed thereon, and a BSA film may be formed thereon. The silane layer facilitates, for example, the formation of an organic film thereon. The ion-exchangeable resin can mitigate noise to measurements by ions. The BSA film can protect the electrode surface from non-specific adsorption of proteins. For example, the protective film may be a polymer and may include a polymer. For example, the polymer of the protective film may be a fluorine-based polymer. The fluorine-based polymer has water repellency and oil repellency, and can prevent or alleviate, for example, non-specific adsorption or contaminants and the like on the electrode.

In some embodiments, the ratio of the amount or amount of modified antigen to the total amount of antigen may be determined. In some embodiments, the amount of the modified protein with respect to the total amount of protein or the ratio of the amounts may be determined. For example, albumin may be used as a protein, and glycated albumin may be used as a modified protein, and the ratio (GA value) thereof may be determined.

In some embodiments, the total amount of antigen may be determined by an optical measurement. In some embodiments, the absorbance of the protein may be measured. In some embodiments, an optical label may be attached to the protein and the optical properties of the optical label may be measured. For example, the absorbance of the optical label may be measured. In some embodiments, the optical label may be a fluorescent label. In some embodiments, fluorescent probes that specifically bind to reagent binding sites may be used. For example, reagents such as dansylamide which binds to the binding site 1 of albumin or BD140 (manufactured by TCI) which binds to the binding site 2 may be used. In some embodiments, the optical label may be an absorbance label. For example, bromocresol green (BCG) and bromocresol purple (BCP) may be used as absorbance labels for albumin.

In some embodiments, an optical measurement for the total amount of antigen may be first performed, and then a measurement of hydrogen peroxide generated by reaction of a substrate with an oxidase via an antigen-antibody complex having an oxidase and a magnetic carrier relative to the modified antigen may be performed. Thereby, for example, it is possible to avoid some effect on the optical measurement by a magnetic substance or oxidase.

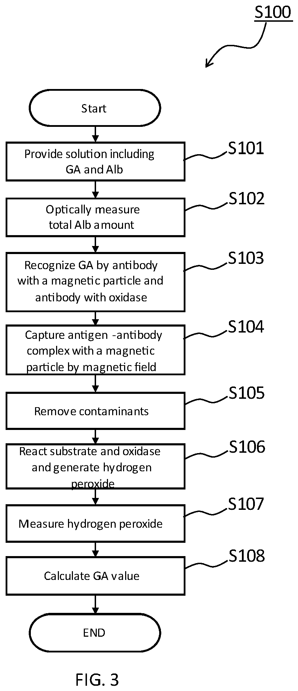

FIG. 3 shows a flow chart of a method for measuring a GA value according to an embodiment (S100).

First, a solution containing non-glycated albumin and glycated albumin (or an albumin solution containing glycated albumin) is prepared (S101).

In some embodiments, the solution may be obtained by collecting bodily fluid. For example, the solution may be collected from tears, saliva, blood, or the like. For example, the solution may be plasma, serum, or the like. The solution may not necessarily contain glycated albumin. Alternatively, a solution containing an amount equal to or lower than the measurement limit even if glycated albumin is contained may be prepared. For example, a solution may be prepared which is considered to contain glycated albumin. Alternatively, a solution may be prepared for the purpose of examining whether or not glycated albumin is contained. Alternatively, a solution may be prepared for the purpose of confirming that glycated albumin is not contained.

In FIG. 3, the amount of total albumin is first measured by optical methods (S102).

Next, an antibody having a magnetic particle and specifically recognizing glycated albumin is introduced into the solution. At the same time, or before and after, an antibody having an oxidase and specifically recognizing glycated albumin is introduced. Thereby, an antigen-antibody complex of glycated albumin in which the oxidase and the magnetic substance are bound is formed (S103).

A magnetic field is applied to the solution in which the antigen-antibody complex has been formed. The antigen-antibody complex that is bound to the magnetic particle is captured in this magnetic field (S104).

In the state where the antigen-antibody complex is captured in the magnetic field, a wash solution is flowed to separate or remove contaminants from the antigen-antibody complex (S105).

A solution containing a substrate (substrate liquid) is introduced into the remaining antigen-antibody complex. The substrate reacts with the oxidase of the antigen-antibody complex and hydrogen peroxide is generated (S106).

By measuring this hydrogen peroxide, the amount of glycated albumin can be determined (S107).

The GA value is determined as the amount of glycated albumin relative to the amount of total albumin determined by S102, i.e. as their ratio.

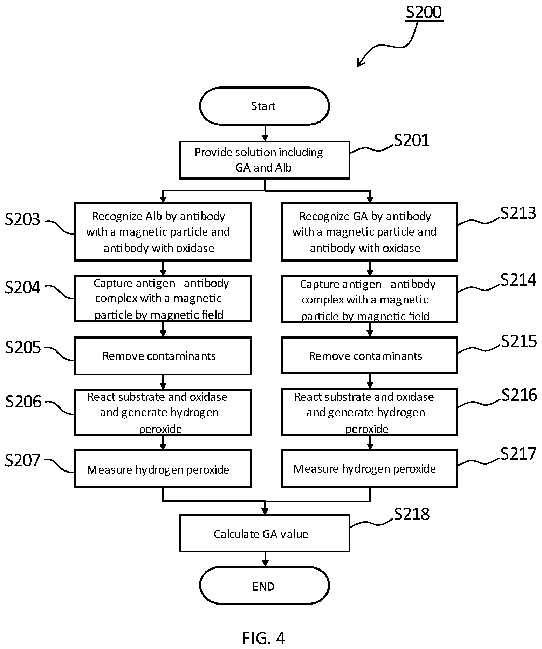

In some embodiments, the both amounts of the antigen and the modified antigen may be determined by measuring the hydrogen peroxide generated by the reaction of the oxidase of the antigen-antibody complex with the substrate. FIG. 4 shows a flow chart of such a method for measuring a GA value according to an embodiment (S200).

First, a solution containing albumin and glycated albumin is provided (S201). The solution is divided for a measurement of total albumin and for a measurement of glycated albumin.

Next, an antibody having a magnetic particle and specifically recognizing albumin is introduced into the solution. At the same time, or before and after, an antibody having an oxidase and specifically recognizing albumin is introduced. Thus, an antigen-antibody complex of albumin in which the oxidase and the magnetic substance are bound is formed (S203).

A magnetic field is applied to the solution in which the antigen-antibody complex has been formed. The antigen-antibody complex that is bound to the magnetic particle is captured in this magnetic field (S1204).

In the state where the antigen-antibody complex is captured in the magnetic field, a wash solution is flowed to separate or remove contaminants from the antigen-antibody complex (S1205).

A solution containing a substrate (substrate liquid) is introduced into the remaining antigen-antibody complex. The substrate reacts with the oxidase of the antigen-antibody complex and hydrogen peroxide is generated (S206).

By measuring this hydrogen peroxide, the amount of albumin can be determined (S207).

Determination of glycated albumin (S213~S217) can be performed in the same manner. That is, an antibody having a magnetic particle and specifically recognizing glycated albumin is introduced into the solution. At the same time, or before and after, an antibody having an oxidase and specifically recognizing glycated albumin is introduced. Thereby, an antigen-antibody complex of glycated albumin in which the oxidase and the magnetic substance are bound is formed (S213).

A magnetic field is applied to the solution in which the antigen-antibody complex has been formed. The antigen-antibody complex that is bound to the magnetic particle is captured in this magnetic field (S214).

In the state where the antigen-antibody complex is captured in the magnetic field, a wash solution is flowed to separate or remove contaminants from the antigen-antibody complex (S215).

A solution containing a substrate (substrate liquid) is introduced into the remaining antigen-antibody complex. The substrate reacts with the oxidase of the antigen-antibody complex and hydrogen peroxide is generated (S216).

By measuring this hydrogen peroxide, the amount of glycated albumin can be determined (S217).

The GA value is determined as the amount of glycated albumin determined by S217 relative to the amount of total albumin determined by S207, i.e. as their ratio.

In some embodiments, with respect to an antigen regardless of whether it is modified or not, an antigen-antibody complex having an oxidase and a magnetic carrier may be formed, and it may be determined by a measurement of hydrogen peroxide generated by reaction of a substrate with an oxidase to the oxidase, and with respect to the modified antigen, an antigen-antibody complex having an oxidase and a magnetic carrier may formed, and it may be determined by a measurement of hydrogen peroxide generated by reaction of a substrate with the oxidase, and the total amount of the antigen regardless of whether or not it is modified and the amount of the modified antigen may be determined. In some embodiments, the total amount of protein regardless of whether glycated or not and the amount of glycated protein may be determined by forming an antigen-antibody complex having an oxidase and a magnetic carrier, respectively, and measuring the hydrogen peroxide by the reaction of the oxidase with the substrate, respectively.

In some embodiments, a solution that is a sample may be divided for a measurement of the total amount of antigen and for a measurement of the amount of modified antigen. For example, a body fluid such as tears, saliva, and blood may be collected, and this may be divided, one of which may be used for the measurement of the amount of glycated protein such as glycated albumin, and the other of which may be used for the measurement of the total amount of protein such as albumin. Thereby, for example, each measurement can be performed on substantially the same sample, and the ratio thereof can be determined more accurately. In some embodiments, the formation of an antigen-antibody complex of an antigen and a modified antigen may be performed substantially simultaneously. For example, the solution may be plasma or serum.

In some embodiments, a solution that is a sample may be divided and each introduced into the same measuring apparatus separately for each measurement. For example, a portion of the solution may be introduced into the measuring apparatus to perform a measurement of the total amount of antigen, which may be discharged after the measurement to wash the inside of the sensor, and then a part of all of the remaining portion of the solution may be introduced into the measuring apparatus to perform the measurement of the amount of the modified antigen. The order of the measurement of the total amount of antigen and the measurement of the amount of modified antigen may be reversed.

### <Example>

In the following, an example of a current measurement of glycated albumin (GA) is shown as one embodiment.

### (1) Preparation of Magnetic Carriers with Primary Antibodies to Capture GA

In this example, magnetic particles (DynabeadsMyOneCarboxylic acid, 1µm in diameter, 10 mg beads/mL, manufactured by Belitas Co., Ltd.) were used as a solid phase, and an anti-GA monoclonal antibody capable of specifically binding GA, which is an antigen, was modified on a surface by the following method to prepare magnetic particles with a primary antibody.
[1] According to the product protocol, a cleaning operation was performed by preparing 200 uL of magnetic particles from a product vial bottle.
[2] 66µL of condensing agent solution (25 mM WSC + 25 mM NHS in MES Buffer) was added to the washed magnetic particles, and the carboxyl groups on the surface of the magnetic particles were activated by rotary stirring for 30 minutes at room temperature.
[3] After removing the excess condensing agent solution in the washing operation, 100 µg of an anti-GA monoclonal antibody dialyzed with a buffer solution was added, and the mixture was diluted up to 100 µL with a buffer solution, followed by a modification reaction on the solid surface by rotary stirring at 37°C for 90 minutes.
[4] After the reaction, a washing operation was performed, and the unreacted activated carboxyl groups left on the surface of the magnetic particles were deactivated by rotary stirring in 200 µL of a buffer (pH 7 to 8) containing 50 mM ethanolamine for 1 hour at room temperature.
[5] After removing the excess reagent and the like in the washing operation, a buffer solution for storage (0.1 % IgG free BSA, 0.05 % Tween-20, and 0.02 % sodium azide in PBS (-)) was added and dispersed, and stored at 4°C.

### (2) Preparation of Enzyme-Labeled Secondary Antibody Solution

Enzyme-labeled secondary antibody solution was prepared by labeling glucose oxidase against anti-human albumin monoclonal antibody using a commercially available labeling kit (Mix-n-Stain TM Glucose Oxidase Antibody Labeling Kit, Biotium).

### (3) Antigen Antibody Reaction

In this example, Lucica (registered trademark) GA-L dedicated GA-L control serum L, H (Asahi Kasei Pharma Co., Ltd.) was used as an antigen solution, and an antigen-antibody reaction was performed by the following procedure to prepare magnetic particles with an antigen-antibody complex.
[1] From the dispersion of magnetic particles with primary antibodies stored at 4°C, 5µL fractions per sample were separated into separate Eppen tubes and washed once with PBS(-) containing 0.1 % Tween-20 and three times with PBS(-).
[2] To each of the washed magnetic particles, 3 µL of each of the 3 kinds of antigen solutions shown in the following table and 10 µL of the enzyme-labeled secondary antibody solution were added, and then the mixture was diluted to 350 µL with PBS (-) containing 4.3 % polyethylene glycol (PEG), and the mixture was subjected to an antigen-antibody reaction by rotary-stirring at 37°C for 30 minutes.

**[Table 1]**

| Unit: g/dL | Blank | Control serum L | Control serum H |
|---|---|---|---|
| GA | 0 | 0.660±0.099 | 1.756±0.176 |
| Albumin | 0 | 4.75±0.48 | 4.76±0.48 |

[3] After the reaction, the unreacted reagent was removed by washing once with PBS (-) containing 0.1 % Tween-20 and three times with PBS (-), and immediately used for preparation of the reaction solution for the measurement.

### (4) Preparation of Reaction Solution for Measurement

The reaction solution for measurement used for the amperometric measurement was prepared according to the following procedure.
[1] To each of the magnetic particles subjected to the antigen-antibody reaction, 100 µL of 5mM glucose PBS (-) solution was added and reacted at room temperature for 5 minutes.
[2] After the reaction, the magnetic particles and the supernatant were separated using a magnet, and the supernatant was collected as a reaction solution for the measurement and stored at 4°C.

### (5) Current Measurement of GA

The current measurement of GA was carried out in real time using an external current measuring apparatus to measure the current flowing into the electrode portion of the electrode tip as an output current. Vref was set at 450 mV, and the current was stabilized by filling the flow path with PBS (-) after the electrode tip was incorporated into the measuring channel jig. After the stabilization, the output current of the reaction solution for measurement was observed by the following procedure, and the current of GA was measured.
[1] 40 µL of the reaction solution for measurement stored at 4°C was flowed into the electrode portion of the electrode tip in the flow path, and the flow of the solution was stopped, and the change in the output current was observed over time.
[2] After about 200 seconds passed, PBS(-) was flowed into the flow path, and the electrode portion was cleaned by pushing the reaction solution for measurement from the electrode portion.
[3] By repeating operation [2] above three times, the output current value was returned to the value at the time of stability.
[4] The above [1] to [3] were performed on the number of samples prepared and the obtained output current values were compared, to estimate the amount of GA present in the antigen solution.

FIG. 5 shows the measurement results of the output current in a graph. The vertical axis indicates the amount of current flowing into the electrode portion of the electrode tip (nA), the horizontal axis indicates the measurement time (sec). The output current once peaked at the start of the measurement (at the time of 10 seconds), then dropped, and became a substantially constant value after a certain time (for example, around 100 seconds). First, at the time of addition (at 10 seconds), a spike-like current flowed. This is considered to be an excessive response due to the disturbance of ions in the liquid in the vicinity of the electrode and the reached hydrogen peroxide, due to the addition of the liquid. Thereafter, as the movement of the liquid settles, the disturbance of the ions on the electrode surface decreases, and at the same time, the number of hydrogen peroxide that was reaching the electrode by multiplying the disturbance decreases. It is thought that as a result, the graph begins to descend. Since hydrogen peroxide is consumed as an oxidation current after reaching the electrode, it can be said that the hydrogen peroxide concentration on the electrode surface is always substantially 0, On the other hand, the hydrogen peroxide concentration in the liquid, which is distant from the electrode, remains high. By this concentration gradient, hydrogen peroxide diffuses from the liquid in the direction of the electrode. The reason why the output current exhibits a constant value after a certain time is considered to be that the consumption of hydrogen peroxide by the electrode and the diffusion of hydrogen peroxide to the electrode reached an equilibrium. As shown in the above graph, it was confirmed that the output current varied depending on the amount of GA present in the antigenic solution, and that the order of the current value was in the order of serum H, serum L, and blank (No-GA), that is, in the order of the concentration of GA.

FIG. 6 shows the average value of the output current in 25 seconds in the graph of FIG. 5, in a bar graph. The vertical axis indicates the value of the output current (nA), and the horizontal axis indicates the name of the antigen solution used. Thus, it was confirmed that the difference in the amount of GA in the antigen solution was electrically distinguished as the difference in the value of the output current even in a short time of only 15 seconds after the measurement reaction solution was added.

In some embodiments, the time profile of the current may be used to determine the amount or concentration of antigen. For example, a current value at a predetermined time may be used, a maximum value of the current value may be used, a change in the current value (e.g., a differential value) may be used, an integral value of the current for a certain time may be used, or these or other current related characteristics may be used.

Calibration of the device may be performed by an extraction inspection for each manufacturing lot. For example, the output characteristics of the extracted devices may be measured and the calibration data may be transmitted to the user of the devices from the same lot or a predetermined lot. In some embodiments, the measurement device may have a calibration flow path (measurement system) in addition to the measurement flow path. For example, a calibration solution containing a predetermined concentration of antigen may be introduced in the calibration channel to measure the concentration thereof. Based on the obtained output, a corresponding calibration curve may be used from among the existing calibration curves.

### <Measurement Device>

The present disclosure includes a measurement device, apparatus, and system.

An apparatus for measuring an antigen according to an embodiment comprises: a first antibody capable of specifically binding to an antigen and bound to a magnetic particle; a second antibody capable of specifically binding to an antigen and bound to an oxidase; a reaction tank for containing an antigen-antibody complex in which the first antibody and the second antibody and an antigen are bound; a magnetic field generator configured to generate a magnetic field in the reaction tank; a substrate liquid tank for containing a substrate liquid including a substrate which reacts with the oxidase, and fluidly connected to the reaction tank in an openable and closable manner; and a hydrogen peroxide sensor for measuring hydrogen peroxide generated by the reaction of the oxidase and the substrate.

In some embodiments, the first antibody and the second antibody may be contained in separate containing portions, respectively. In some embodiments, the first antibody and the second antibody may be contained in a containing portion separate from the reaction tank in which the antigen is recognized. In some embodiments, the first antibody and the second antibody may be contained in the reaction tank in which the antigen is recognized.

In some embodiments, the antigen-antibody complex may be captured in a magnetic field in the reaction tank. In some embodiments, the antigen-antibody complex may be captured in a magnetic field at a location outside the reaction tank. In some embodiments, the antigen-antibody complex may be captured in a magnetic field. In some embodiments, the antigen-antibody complex may be captured outside of the space in which the magnetic field acts.

In some embodiments, a substrate liquid tank may be fluidly coupled in an openable and closable manner to a section in which an antigen-antibody complex is being captured. In some embodiments, the antigen-antibody complex is captured in a magnetic field in a reaction tank, and a substrate liquid tank may be fluidly coupled in an openable and closable manner to the reaction tank.

In some embodiments, a hydrogen peroxide sensor may be disposed in the reaction tank. In some embodiments, a hydrogen peroxide sensor may be located outside the reaction tank.

In some embodiments, the measurement system may further comprise a buffer tank. The buffer tank may be fluidly connected in an openable and closable manner to a space containing a physiological buffer or a reaction tank in which an antigen-antibody complex is being captured. The buffer may be used, for example, to flow into a reaction tank or the like to separate or pour out contaminants and the like that affect the measurement from the captured antigen-antibody complex.

In some embodiments, the measurement system may comprise an inlet for introducing a solution including an antigen. In some embodiments, the measurement system may comprise a drain tank to contain a substrate liquid, a buffer, or the like after use. For example, the drain tank may be fluidly connected to a reaction tank or a space in which an antigen-antibody complex is being captured. The drain tank may have an air hole for removing air. Excess solution can be discharged and hydrogen peroxide required for measurement can be efficiently separated.

FIG. 7 schematically shows a configuration of a measurement system 100 according to an embodiment. In the reaction tank 130, a first antibody 110 having a magnetic and a second antibody 120 having an oxidase are contained. Prior to use, these antibodies are stored in the reaction tank 130 in a dry state. The reaction tank 130 has an inlet 131 of a solution, and from the inlet 131 a solution 101 containing an antigen 102 as measurement target, a contaminant 103, and the like is introduced into the measurement system 100 or the reaction tank 130. The antigen 102 contained in the solution 101 is recognized by the first antibody 110 and the second antibody 120, and an antigen-antibody complex (not shown) is formed in the reaction tank.

External to the reaction tank 130 is an electromagnet 150, which generates a magnetic field in the reaction tank 130 and captures the antigen-antibody complex in the reaction tank 130. The magnetic field is controlled by turning on and off the current to the electromagnet 150. The control of the current to the electromagnets 150 is controlled by an external CPU 160.

A buffer tank 180 containing a buffer liquid 181 is fluidly connected in an openable and closable manner to the reaction tank 130. When the buffer liquid 181 is introduced from the buffer tank 180 to the antigen-antibody complex captured by a magnetic field, contaminants 103 and the like which are not bound to the magnetic substance are pushed out of the reaction tank 130. The solution pushed out enters the drain tank 190 fluidly coupled to the reaction tank 130.

A substrate liquid tank 170 containing a substrate liquid 171 is fluidly connected in an openable and closable manner to the reaction tank 130. When a substrate liquid 171 is introduced from the substrate liquid tank 170 to the antigen-antibody complex captured in a magnetic field in the reaction tank 130, a substrate contained in the substrate liquid 171 catalyzes and reacts with an oxidase of the antigen-antibody complex to generate hydrogen peroxide.

A hydrogen peroxide electrode 140 is disposed in the reaction tank 130. In FIG. 7, the hydrogen peroxide electrode 140 is coated with a protective film 141 to inhibit or reduce contaminants from adsorbing non-specifically with the electrode. Hydrogen peroxide generated in the reaction tank 130 is detected by the hydrogen peroxide electrode 140, and an output current is measured. The output current from the hydrogen peroxide electrode 140 is amplified, converted into a voltage, and measured. Measured current value and other calculations are performed in CPU 160.

After the measurement, the electromagnet 150 is switched off to dissipate the magnetic field, and the substances in the reaction tank 130 including the antigen-antibody complex can be flowed into the drain tank 190.

FIG. 8 schematically shows a configuration of a measurement system 200 according to an embodiment. In the measurement system 200 shown in FIG. 8, a hydrogen peroxide electrode 240 is disposed in a measurement tank 290 separately configured from the reaction tank 230. The measurement tank 290 is fluidly coupled to the reaction tank 230. The antigen-antibody complex of the antigen and the antibody 210,220 is retained in the reaction tank, and the generated hydrogen peroxide can be measured in the separately configured measurement tank 290. Thus, for example, by reducing the concentration of contaminants which may affect the hydrogen peroxide electrode 240 and the risk of contact of the magnetic substance with the electrode surface, and also controlling the catalytic reaction time to the substrate, for example, by an oxidase, the drift current derived from the hydrogen peroxide generated during the measurement can be reduced, and the measurement of the hydrogen peroxide can be performed with relatively high accuracy.

In some embodiments, the measurement system may be configured of a plurality of components. For example, the measurement system may be configured of a measurement cassette (measurement sensor, measurement cartridge, measurement device, etc.) having a flow path system from the introduction of the sample to the drain and a main body having a part or all of the rest.
The measurement cassette may be configured so as to be coupled to the main body. The measurement cassette may be configured to be coupled to the main body either electrically, magnetically, mechanically, or otherwise or by any combination thereof. For example, a magnetic field generator (magnet, permanent magnet, electric magnet, etc.) may be disposed to the main body. For example, a mechanism (a mechanical apparatus and the like) for controlling the delivery of liquid in the measurement cassette may be disposed to the main body. For example, an optical measurement system may be disposed in the body. For example, a solution used for measurement, liquid feeding, or the like, or a tank thereof (solution tank) may be disposed in the measurement cassette, and may be configured to be disposed on the main body side and introduced into the measurement cassette if necessary. Components that are used repeatedly or multiple times may be disposed in the main body, and disposable configurations may be disposed in the measurement device. This makes it possible, for example, to miniaturize the measurement device and make it easier to use it. In some examples, the liquid feeding may be performed by applying pressure to the flow path from the outside or by introducing air or gas into the flow path. In some examples, the apparatus may be equipped with a liquid feeding mechanism, a liquid feeding portion, a liquid feeding means, a pressure mechanism, a pressure air introduction unit or mechanism to perform a part or all of them.

In some embodiments, the measurement system may be configured with a hydrogen peroxide sensor, such as an electrode, and other sensors. In some embodiments, the measurement system may have a plurality of hydrogen peroxide sensors. This makes it possible, for example, to measure a plurality of types of measurement targets and/or to perform a plurality of measurements.

For example, the measurement system may have a hydrogen peroxide sensor and an optical system. Both electrical and optical measurements can be performed. The optical measurement may be, for example, an absorbance measurement or a fluorescence measurement. For example, the hydrogen peroxide sensor may be used to electrically measure glycated protein (e.g., GA), and a total amount of a protein (e.g., albumin) may be optically measured regardless of whether glycated or not.

FIG. 9 schematically shows a configuration of a measurement cassette (device) 300 according to an embodiment. FIG. 9A shows a plan view of the measurement cassette 300, and FIG. 9B shows a cross-sectional view of A-A in FIG. 9A. The measurement cassette 300 is inserted into and fixed to the slit of the measurement instrument main body 350. The electrode terminal 341 of the hydrogen peroxide sensor 340 is in electrical contact with the electrode terminal 354 of the main body 350 to enable electrical signals from the sensor 340 to be transmitted to the main body 350.

The measurement cassette 300 shown in FIG. 9A has an inlet 331 for the solution from which the solution can be introduced into the measurement cassette 300. For example, a body fluid such as tears or saliva are collected, and absorbance labeling reagents such as BCP are mixed thereto. Absorbent labeling reagents bind to protein contained in the collected solution. A body fluid or a solution containing the body fluid after reaction with the absorbance labeling reagent is introduced into the inlet 331. The inlet 331 may be configured to be sealed after the introduction of the solution.

From the inlet 331, the flow path passes through an optical measurement channel portion 332. The optical measurement flow path portion 332 is formed in a direction connecting both sides of the measurement cassette, the end may be sealed with a light window so that light can travel through. The measurement instrument main body 350 and the measurement cassette 300 may be positioned with sufficient accuracy. For example, the actual optical path defined by the light emitter 352a and the light receiver 352b may be positioned sufficiently well in terms of the measurement accuracy with respect to the predetermined optical path of the optical measurement flow path section 332. Light emitted from the light emitter 352a provided on the outside or the main body enters the inside from one end, passes through the optical measurement channel portion 332, exits from the opposite end, and can be detected by the light receiver (optical sensor) 352b for optical characteristics such as intensity, absorbance, fluorescence output, and the like.

The solution which has passed through the optical measurement channel portion 332 is then introduced into the reaction tank 330. In the reaction tank 330, an antibody material 310 containing a first antibody and a second antibody is disposed. The antigen contained in the solution and this antibody material 310 come into contact with each other to form an antigen-antibody complex by an antigen-antibody reaction.

The solution in the reaction tank 330 is compressed from the outside, and the compression thereof can be repeated. In FIG. 9, a part of the reaction tank 330 can be elastically deformed (at a region surrounded by a dotted line). A compression mechanism 353 is disposed to the main body 350 and can push the reaction tank 330. Thus, for example, the solution can be efficiently mixed, and for example, the antigen-antibody reaction can be promoted.

After the antigen-antibody reaction has been sufficiently performed, a magnetic field can be generated in the reaction tank 330 using a magnet 351. A magnetic field may be generated to cover the portion where the antibody 310 is disposed. The magnetic field allows the antigen-antibody complex to be captured in the magnetic field along with the magnetic material attached to one of the antibodies 310.

In some embodiments, for example as shown in FIG. 9B, one magnet 351 may be disposed to each of the reaction tank 330 and the measurement cassettes 300. In some embodiments, a pair of magnets may be disposed. In some embodiments, a pair of magnets may be operated alternately. By operating the magnets alternately, the direction of the magnetic field may be changed. The direction of the magnetic field may be changed at intervals of several seconds, several tens of seconds, 30 seconds, 1 minute, or the like. By changing the direction of the magnetic field, for example, the mixing of the solution can be promoted and the desired reaction can be promoted.

A tank 380 containing a washing buffer and a tank 370 containing glucose are fluidly connected between the optical measurement channel portion 332 and the reaction tank 330.

After the antigen-antibody complex having the magnetic substance is captured in the magnetic field, the buffer tank 380 is pushed by the compression mechanism 353, and the buffer is sent to the reaction tank 330. Contaminants in the reaction tank 330 can be flowed out by the buffer. The pushed-out solution passes through the measurement tank 340 and is collected in the drain tank 390 in FIG. 9A.

Since the inlet 331 is sealed and the drain tank 390 is provided with an air hole, the solution can flow from the inlet 331 to the drain tank 390 under the internal positive pressure.

Next, the glucose tank 370 is compressed by the compression mechanism 353, and the glucose solution is poured into the reaction tank 330. Glucose reacts with the oxidase of the antigen-antibody complex to generate hydrogen peroxide.

Hydrogen peroxide generated in the reaction tank 330 enters and is measured in the measurement tank 340 with a hydrogen peroxide sensor by diffusion in the solution or compression of the reaction tank 330 by a compression mechanism 353. The electrical signal from the hydrogen peroxide sensor is transmitted to the measurement instrument main body 350 via terminals 341, 354.

FIG. 10 schematically shows a configuration of a measurement cassette (device) 400 according to an embodiment. It is a partially modified embodiment of the measurement cassette 300 shown in FIG. 9. The measurement cassette (device) of FIG. 10 has an optical label (absorbance label or fluorescent label) reagent in the same cassette and can react a body fluid with an optical label reagent in the cassette.

In FIG. 10, an optical labeling solution tank 460 is connected to an inlet 431, is compressed by a compression mechanism (not shown) of the main body, and the internal solution is introduced into the flow path system through the inlet 431. The inlet 431 may be sealable by a sealing lid (not shown) or the like.

FIG. 11 schematically shows a configuration of a measurement cassette 500 according to an embodiment. It is a partially modified embodiment of the measurement cassette 300 shown in FIG. 9. Antibody 510 is disposed not in a reaction tank 530 but before that, and after the optical measurement channel 532. The reaction tank 530 may be configured so as to be compressed by a compression mechanism (not shown) so as to be able to mix the solution inside well.

FIG. 12 schematically shows a configuration of a measurement cassette (device) 400 according to an embodiment. It is a partially modified embodiment of the measurement cassette 300,400 shown in FIG. 9 or FIG. 10. The measurement cassette (device) 600 shown in FIG. 12 does not have the washing buffer tank 380, the glucose tank 370 of the measurement cassette 300 shown in FIG. 9, and the optical labeling solution tank 460 of the measurement cassette 400 shown in FIG. 10. From outside, for example from a tank disposed to the main body (not shown), the liquid or solution in them is provided to the measurement cassette (device) 600 of FIG. 12. Specifically, the measurement cassette 600 of FIG. 12 has a solution inlet 631, an optical label solution inlet 660, a cleaning solution inlet 680 and a glucose inlet 670. A measurement cassette 600 is mounted on the main body (not shown) and each inlet is fluidly connected to a corresponding solution tank (not shown). The corresponding solution from each solution tank is introduced into the flow path in the measurement cassette 600 from each reagent inlet 660,680,670 at a necessary or predetermined timing and amount.

In some embodiments, the measurement system may have a plurality of channel systems having hydrogen peroxide sensors. In some embodiments, the measurement system may have a pair of flow path systems with hydrogen peroxide sensors. For example, in one channel system, a hydrogen peroxide sensor may be used to electrically measure a glycated protein (e.g., GA), and in another channel system, a hydrogen peroxide sensor may be used to measure a total amount of a protein (e.g., albumin) regardless of whether it is glycated or not.

FIG. 13 schematically shows a configuration of a measurement cassette (device) 700 according to an embodiment. The measurement cassette 700 of FIG. 13 has a pair of measurement flow path systems 700a,700b, such as the measurement cassette 400 of FIG. 10. In FIG. 13, the pair of measurement flow path systems 700a,700b are depicted symmetrically with respect to each other, but may not be symmetrical, may be asymmetrical, or may have different configurations. The measurement channel systems 700a,700b each have a solution (sample) inlet 731a,731b.

In some embodiments, each of the plurality of measurement channel systems may have a solution inlet. In some embodiments, the measuring device may have one solution inlet, from which to feed the solution inlet to each of the plurality of measuring channel systems. In some embodiments, the measuring device may have a plurality of solution inlets. The number of measurement channel systems and the number of solution inlets may be different.

The same measurement may be performed on different substances between the measurement channel systems 700a,700b, or different measurements may be performed. In some embodiments, the antibody materials 710a,710b disposed in the measurement flow path systems 700a,700b may be substantially the same. In some embodiments, the antibody materials 710a,710b disposed in the measurement flow path systems 700a, 700b may comprise different antibodies. For example, the amount of glycated protein (e.g., GA) may be measured in the measurement channel system 700a, and the total amount of protein (e.g., the total amount of albumin) may be measured in the measurement channel system 700b. In that case, for example, the antibody material 710a may include an anti-GA antibody and, for example, the antibody material 710b may include an anti-Alb antibody.

FIG. 14 schematically shows a configuration of a measurement cassette (device) 800 according to an embodiment. It is a partially modified embodiment of the measurement cassette 700 shown in FIG. 13. The measurement cassette (device) 800 of FIG. 14 does not have a wash buffer tank, a glucose tank, or an optical labeling solution tank therein. The measurement cassette 800 of FIG. 14 has sample (solution) inlets 831a,831b and reagent (solution) inlets 860a,860b fluidly coupled thereto, for the measurement channel systems 800a,800b, respectively. Through these reagent (solution) inlets 860a,860b, a reagent (solution) is provided in the measurement cassette (device) 800 of FIG. 14 from outside, for example from a tank disposed to the main body (not shown).

The measurement cassette 800 of FIG. 14 has a pair of measurement flow path systems 800a,800b, such as the measurement cassette 600 of FIG. 12. In FIG. 14, the pair of measurement flow path systems 800a,800b are depicted symmetrically with respect to each other, but may not be symmetrical, may be asymmetrical, and may have different configurations.

Some embodiments of the present disclosure can be used for measurements of glycated proteins. Glycated proteins have been used as a diagnostic indicator of diabetes and as a determinant of daily glycemic management, along with their measured blood glucose levels. However, because the blood glucose level measurement reflects the instantaneous blood glucose level, it is easily changed by the influence of the prior meal, medication, etc., and is insufficient from the viewpoint of the management index of the lifestyle for the diabetes mellitus treatment. Glycated proteins, on the other hand, are a stable indicator because they reflect longer-term mean blood glucose levels, such as the past few weeks to months. For example, glycated albumin (GA) and glycated hemoglobin (HbA1c) are valuable as judgment indicators because the ratio of glycated protein per protein (GA/albumin, or HbA1c/hemoglobin) is measured, and therefore is less likely to differ from person to person and to be influenced by protein concentrations. However, HbAlc levels reflect mean blood glucose levels over the last 1 to 2 months, and take too longer to gives feeling of the benefits from life style improvements. On the other hand, since GA is a value reflecting the mean blood sugar level in the last two weeks, GA can obtain a result in a shorter time than HbA1c, and therefore GA is attracting attention as a more efficient control marker of blood sugar level.

In order to measure the GA value, it was necessary to carry out a HPLC method combining ionexchange columns and affinity adsorption to boronic acid. However, there were problems that the measurement requires a large and expensive dedicated instrument and a long time, and since then various measurement methods such as an enzymatic method and an immunological method have been developed, whereby the GA measurement can be performed more easily and in a shorter time than the conventional HPLC method. Yet, because optical apparatuses are required for these measurements, the apparatuses have not been downsized, and they cannot be said to be optimal as a method for easily performing GA measurements in daily life.

According to some embodiments of the present disclosure, an instrument that measures GA or GA values can be manufactured relatively inexpensively or miniaturized.

The present disclosure also includes the following embodiments:
A01
   A method for measuring an antigen, comprising:
   providing a solution containing an antigen;
   providing a first antibody that specifically recognizes the antigen and is bound to a magnetic carrier;
   providing a second antibody that specifically recognizes the antigen and is modified with an oxidase;
   providing (a substrate liquid including) a substrate which reacts with the oxidase;
   allowing the first antibody to recognize the antigen;
   allowing the second antibody to recognize the antigen;
   using a magnetic field to capture an antigen-antibody complex of the antigen recognized by the first antibody and the second antibody in the magnetic field;
   washing the antigen-antibody complex while it is captured in the magnetic field;
   reacting the substrate with the antigen-antibody complex to produce hydrogen peroxide; and
   measuring the hydrogen peroxide.
A02
   The method of embodiment A01, further comprising providing a hydrogen peroxide sensor; wherein said capturing the antigen-antibody complex in the magnetic field includes capturing the antigen-antibody complex in the vicinity of the hydrogen peroxide sensor by the magnetic field.
A03
   The method of embodiment A01, further comprising: separating the generated hydrogen peroxide out of the magnetic field while capturing the antigen-antibody complex in the magnetic field using the magnetic field.
A04
   The method of embodiment A02 or A03, wherein the antigen as measurement target includes a modified protein.
A05
   The method of embodiment A04, wherein the modified protein includes a glycosylated protein.
A06
   The method of embodiment A05, wherein at least one of the first antibody and the second antibody includes a monoclonal antibody to the glycated protein as measurement target substance.
A07
   The method of embodiment A06, wherein said at least one of the first antibody and the second antibody includes a monoclonal antibody that recognizes a glycation site of the glycated protein.
A07b
   The method of embodiment A06, wherein said at least one of the first antibody and the second antibody includes a monoclonal antibody to the glycated protein as measurement target.
A07c
   The method of embodiment A07 or A07b, wherein the glycation site of the glycated protein recognizes a different feature of the glycated protein than the non-glycated protein.
A08
   The method of any one of embodiments A05 to A07c, wherein one of the first antibody and the second antibody is a monoclonal antibody that recognizes the glycated protein, and the other of the first antibody and the second antibody is a polyclonal antibody which recognizes the glycated protein.
A09
   The method of any one of embodiments A01 to A08, wherein the oxidase includes a glucose oxidase, and the substrate includes glucose.
A11
   The method of any one of embodiments A01 to A09, wherein said measuring the hydrogen peroxide includes using a hydrogen peroxide electrode.
A12
   The method of any one of embodiments A01 to A11, wherein said measuring the hydrogen peroxide includes:
   measuring a current at the hydrogen peroxide electrode, and
   measuring a concentration of the antigen in the solution from the current.
A13
   The method of embodiment A11 or A12, wherein the hydrogen peroxide electrode has a thin film formed on its surface to reduce the influence of contaminants.
A14
   The method of embodiment A13, wherein the hydrogen peroxide electrode is covered with a protective film substantially consisting of a polymer.
A21
   The method of any one of embodiments A01 to A03, wherein the antigen includes glycated albumin.
A22
   The method of embodiment A21, wherein the solution including the glycated albumin includes a bodily fluid derived from a living body.
A23
   The method of embodiment A22, wherein the body fluid is tear or saliva.
A23b
   The method of embodiment A22, wherein the bodily fluid is blood, plasma or serum.
A24
   The method of any one of embodiments A21 to A23B, wherein the glycated albumin is such that one of the first antibody and the second antibody is an anti-GA monoclonal antibody, and the other of the first antibody and the second antibody is an anti-albumin antibody.
A101
   A method for measuring a GA value, comprising:
   providing a solution including glycated albumin (glycoalbumin) and non-glycated albumin;
   measuring the total amount of albumin including the glycated albumin and the non-glycated albumin;
   providing a first glycated albumin antibody which specifically recognizes the glycated albumin and is bound to a magnetic carrier,
   providing a second glycated albumin antibody which specifically recognizes the glycated albumin and is modified with an oxidase;
   providing (a substrate liquid including) a substrate which reacts with the oxidase;
   allowing the first glycated albumin antibody to recognize the glycated albumin;
   allowing the second glycated albumin antibody to recognize the glycated albumin;
   using a magnetic field to capture an antigen-antibody complex of the glycated albumin recognized by the first glycated albumin antibody and the second glycated albumin antibody; washing the antigen-antibody complex of the glycated albumin while it is captured in the magnetic field;
   reacting the substrate with the antigen-antibody complex to produce hydrogen peroxide; measuring the hydrogen peroxide; and
   obtaining the ratio (GA value) between the amount of the glycated albumin and the amount of the total albumin from the measured amount of the hydrogen peroxide and the measured total amount of the albumin.
A102
   The methods of embodiment A101, wherein said measuring the total amount of albumin including the glycated albumin and the non-glycated albumin includes optically measuring the albumin.
A103
   The method of embodiment A102, wherein said optically measuring the albumin includes binding an absorbance labeling reagent to the albumin.
A104
   The method of embodiment A103, wherein the absorbance labeling reagent is bromocresol green (BCG) or bromocresol purple (BCP).
A105
   The method of embodiment A103 or A104, wherein said optically measuring the albumin includes measuring the absorbance of the absorbance labeling reagent bound to the albumin.
A106
   The method of any one of embodiments A101 to A105, wherein said optically measuring the albumin is performed prior to allowing the first glycated albumin antibody to recognize the glycated albumin and allowing the second glycated albumin antibody to recognize the glycated albumin.
A201
   A method for measuring glycated albumin (GA), comprising:
   providing a solution including an antigen;
   providing a first albumin antibody that specifically recognizes the albumin and is bound to a magnetic carrier;
   providing a second albumin antibody that specifically recognizes the albumin and is modified with a second oxidase;
   providing (a substrate liquid including) a second substrate which reacts with the second oxidase;
   allowing the first albumin antibody to recognize the albumin;
   allowing the second albumin antibody to recognize the albumin;
   using a magnetic field to capture an antigen-antibody complex of the albumin recognized by the first albumin antibody and the second albumin antibody in the magnetic field;
   washing the albumin antigen-antibody complex while it is captured in the magnetic field; reacting the second substrate with the antigen-antibody complex of the albumin recognized by the first albumin antibody and the second albumin antibody to produce second hydrogen peroxide;
   measuring the second hydrogen peroxide;
   providing a first glycated albumin antibody that specifically recognizes the glycated albumin included in the albumin and is bound to a magnetic carrier;
   providing a second glycated albumin antibody that specifically recognizes the glycated albumin and is modified with a first oxidase;
   providing (a substrate liquid including) a first substrate which reacts with the first oxidase;
   allowing the first glycated albumin antibody to recognize the glycated albumin;
   allowing the second glycated albumin antibody to recognize the glycated albumin;
   using a magnetic field to capture an antigen-antibody complex of the glycated albumin recognized by the first glycated albumin antibody and the second glycated albumin antibody in the magnetic field;
   washing the glycated albumin antigen-antibody complex while it is captured in the magnetic field;
   reacting the first substrate with the antigen-antibody complex of the glycated albumin recognized by the first glycated albumin antibody and the second glycated albumin antibody to produce first hydrogen peroxide;
   measuring the first hydrogen peroxide; and
   obtaining the ratio (GA value) between the amount of the glycated albumin and the amount of total albumin from the measured amount of the first hydrogen peroxide and the measured amount of the second hydrogen peroxide.
B01
   An apparatus for measuring a glycated protein, comprising
   an inlet for introducing a solution including a glycated protein;
   a reaction tank for containing a first antibody capable of specifically binding to the glycated protein and bound to a magnetic particle and a second antibody capable of specifically binding to the glycated protein and bound to a glucose oxidase, and for containing an antigen-antibody complex obtained by reacting the first antibody and the second antibody with the glycated protein;
   a magnetic field generator configured to generate a magnetic field in the reaction tank;
   a substrate liquid tank for containing a substrate liquid including a glucose which reacts with the glucose oxidase, and being fluidly connected in an openable and closable manner to the reaction tank; and
   a hydrogen peroxide electrode for measuring hydrogen peroxide generated from the glucose oxidase.
B02
   The measuring apparatus of embodiment B01, further comprising a buffer tank containing a buffer and fluidly connected in an openable and closable manner to the reaction tank.
B03
   The measuring apparatus of embodiment B02, wherein the buffer tank is configured to separate contaminants from the antigen-antibody complex by introducing the buffer from the buffer tank into the reaction tank after the antigen-antibody complex is formed.
B04
   The measuring apparatus of embodiment B02 or B03, further comprising a hydrogen peroxide measurement tank containing the hydrogen peroxide electrode and fluidly connected in an openable and closable manner to the reaction tank.
B05
   The measuring apparatus of embodiment B04, wherein the buffer tank is configured to send the generated hydrogen peroxide to the hydrogen peroxide measurement tank by introducing the buffer from the buffer tank into the reaction tank, after the hydrogen peroxide is generated,
B06
   The measuring apparatus of any one of embodiments B01 to B05, further comprising a drain tank configured to contain a solution discharged from the reaction tank.
B07
   The measuring apparatus of any one of embodiments B01 to B06, further comprising a liquid feeding mechanism for feeding the solution in the apparatus.
B08
   The measuring apparatus of embodiment B07, wherein the liquid feeding mechanism enables liquid feeding by air pressure or external pressure.
B101
   An apparatus for measuring an antigen, comprising:
   an inlet for introducing a solution including an antigen;
   a first antibody capable of specifically binding to the antigen and bound with a magnetic particle;
   a second antibody capable of specifically binding to the antigen and bound to an oxidase;
   a reaction tank for containing an antigen-antibody complex obtained by reacting the first antibody and the second antibody with the antigen;
   a magnetic field generator configured to generate a magnetic field in the reaction tank;
   a substrate liquid tank containing a substrate liquid including a substrate which reacts with the oxidase, and being fluidly connected in an openable and closable manner with the reaction tank; and
   a hydrogen peroxide sensor for measuring hydrogen peroxide generated from the reaction of the oxidase and the substrate.
B102
   The measuring apparatus of embodiment B 101, wherein the reaction tank contains a first antibody which is capable of specifically binding to the glycated protein and bound to a magnetic particle, and a second antibody which is capable of specifically binding to the glycated protein and bound with a glucose oxidase.
B103
   The measuring apparatus of embodiment B 102, further comprising a buffer tank containing a buffer and fluidly connected in an openable and closable manner to the reaction tank.
B104
   The measuring apparatus of embodiment B103, wherein the buffer tank is configured to separate contaminants from the antigen-antibody complex by introducing the buffer from the buffer tank into the reaction tank after the antigen-antibody complex is formed.
B105
   The measuring apparatus of embodiment B103 or B104, further comprising a hydrogen peroxide measurement tank containing the hydrogen peroxide sensor and fluidly connected in an openable and closable manner to the reaction tank.
B106
   The measuring apparatus of embodiment B105, wherein the buffer tank is configured to send the generated hydrogen peroxide to the hydrogen peroxide measurement tank by introducing the buffer from the buffer tank into the reaction tank, after the hydrogen peroxide is generated.
B107
   The measuring apparatus of embodiment B106, further comprising a drain tank configured to contain a solution discharged from the hydrogen peroxide measurement tank.
B108
   The measuring apparatus of embodiment B103 or B104, wherein the hydrogen peroxide sensor is disposed in the reaction tank.
B109
   The measuring apparatus of embodiment B108, further comprising a drain tank configured to contain a solution discharged from the reaction tank.
B110
   The measuring apparatus of any one of embodiments B101 to B 109, wherein the hydrogen peroxide sensor includes a hydrogen peroxide electrode.

While several embodiments and examples of the present disclosure have been described above, these embodiments and examples illustrate the present disclosure. For example, each of the embodiments described above has been described in detail in order to explain the present disclosure in an easy-to-understand manner, and dimensions, configurations, materials, and circuits may be additionally changed as necessary. Embodiments in which one or more features of the present disclosure described above are arbitrarily combined are also included in the scope of the present disclosure. It is intended that the appended claims cover numerous modifications to the embodiments without departing from the spirit and scope of the present disclosure. Accordingly, the embodiments and examples disclosed herein have been shown by way of illustration and should not be considered as limiting the scope of the present disclosure.

### DESCRIPTION OF REFERENCES

- 1: Antigen solution
- 2: Glycated protein
- 3: Sugar
- 4: Protein
- 5: Contaminant
- 10: Antibody having a magnetic particle
- 11: Antibody
- 12: Magnetic particle
- 20: Antibody with oxidase
- 21: Antibody
- 22: Oxidase
- 30: Antigen-antibody complex
- 40: Wash solution
- 50: Substrate
- 100: Measuring system
- 101: Solution
- 102: Antigen
- 103: Contaminant
- 110: First antibody having magnetism
- 120: Second antibody with oxidase
- 130: Reaction tank
- 140: Hydrogen peroxide electrode
- 141: Protective film
- 150: Electromagnet
- 160: CPU
- 170: Substrate liquid tank
- 171: Substrate liquid
- 180: Buffer tank
- 181: Buffer solution
- 190: Drain tank
- 200: Measuring system
- 210,220: Antibody
- 230: Reaction tank
- 240: Hydrogen peroxide electrode
- 290: Measurement tank
- 300: Measurement cassette (device)
- 310: Antibody materials including first and second antibodies
- 330: Reaction tank
- 331: Solution inlet
- 332: Optical measurement channel portion
- 340: Hydrogen peroxide sensor / measurement tank
- 341: Electrode terminal
- 350: Measuring instrument main body
- 351: Magnet
- 352a: Light emitter
- 352b: Light receiver
- 353: Compression mechanism
- 354: Electrode terminal
- 370: Glucose solution tank
- 380: Buffer tank
- 390: Drain tank
- 400: Measurement cassette
- 431: Solution inlet
- 460: Optical labeling solution tank
- 500: Measurement cassette
- 510: Antibody
- 530: Reaction tank
- 532: Optical measurement channel
- 600: Measurement cassette
- 631: Solution inlet
- 660,680,670: Reagent inlet
- 700: Measurement cassette
- 700a, 700b: Measurement flow channel system
- 731a, 731b: Solution (sample) inlet
- 710a, 710b: Antibody material
- 800: Measurement cassette
- 800a, 800b: Measurement channel system
- 831a, 831b: Solution (sample) inlet
- 860a, 860b: Reagent (solution) inlet

## Claims

1. A method for measuring antigens, comprising:
providing a solution containing an antigen;
providing a first antibody that specifically recognizes the antigen and is bound to a magnetic carrier;
providing a second antibody that specifically recognizes the antigen and is modified with an oxidase;
providing a substrate which reacts with the oxidase;
allowing the first antibody to recognize the antigen;
allowing the second antibody to recognize the antigen;
using a magnetic field to capture an antigen-antibody complex of the antigen recognized by the first antibody and the second antibody in the magnetic field;
washing the antigen-antibody complex while it is captured in the magnetic field;
reacting the substrate with the antigen-antibody complex to produce hydrogen peroxide; and
measuring the hydrogen peroxide.

2. The method of Claim 1, further comprising providing a hydrogen peroxide sensor;
wherein said capturing the antigen-antibody complex in the magnetic field includes capturing the antigen-antibody complex in the vicinity of the hydrogen peroxide sensor by the magnetic field.

3. The method of Claim 1, further comprising: separating the generated hydrogen peroxide out of the magnetic field while capturing the antigen-antibody complex in the magnetic field using the magnetic field.

4. The method of Claim 2 or 3, wherein the antigen as measurement target comprises a modified protein.

5. The method of Claim 4, wherein the modified protein includes a glycosylated protein.

6. The method of Claim 5, wherein at least one of the first antibody and the second antibody includes a monoclonal antibody to the glycated protein as measurement target.

7. The method of Claim 6, wherein said at least one of the first antibody and the second antibody includes a monoclonal antibody that recognizes a glycation site of the glycated protein.

8. The method of Claim 6 or 7, wherein said at least one of the first antibody and the second antibody includes a monoclonal antibody which recognizes a conformation generated by glycation of the protein.

9. The method of any one of Claims 5 to 8, wherein one of the first antibody and the second antibody is a monoclonal antibody that recognizes the glycated protein, and
the other of the first antibody and the second antibody is a polyclonal antibody which recognizes the glycated protein.

10. The method of any one of Claims 1 to 9, wherein the oxidase includes a glucose oxidase, and the substrate includes glucose.

11. The method of any one of Claims 1 to 10, wherein said measuring the hydrogen peroxide includes using a hydrogen peroxide electrode.

12. The method of any one of Claims 1 to 11, wherein said measuring the hydrogen peroxide includes:
measuring a current at the hydrogen peroxide electrode, and
measuring a concentration of the antigen in the solution from the current.

13. The method of Claim 11 or 12, wherein the hydrogen peroxide has a thin film formed on its surface to reduce the influence of contaminants.

14. The method of Claim 13, wherein the hydrogen peroxide electrode is covered with a protective film substantially consisting of a polymer.

15. The method of any one of Claims 1 to 14, wherein the antigen includes glycated albumin.

16. The method of Claim 15, wherein the solution including the glycated albumin includes a bodily fluid derived from a living body.

17. The method of Claim 16 wherein the body fluid is tear or saliva.

18. The method of Claim 16, wherein the bodily fluid may be blood, plasma or serum.

19. The method of any one of Claims 15 to 18, wherein the glycated albumin is such that one of the first antibody and the second antibody is an anti-GA monoclonal antibody, the other of the first antibody and the second antibody is an anti-albumin antibody.

20. A method for measuring a GA value, comprising:
providing a solution including glycated albumin and non-glycated albumin;
measuring the total amount of albumin including the glycated albumin and the non-glycated albumin;
providing a first glycated albumin antibody which specifically recognizes the glycated albumin and bound to a magnetic carrier;
providing a second glycated albumin antibody which specifically recognizes the glycated albumin and is modified with an oxidase;
providing a substrate which reacts with the oxidase;
allowing the first glycated albumin antibody to recognize the glycated albumin;
allowing the second glycated albumin antibody to recognize the glycated albumin,
using a magnetic field to capture an antigen-antibody complex of the glycated albumin recognized by the first glycated albumin antibody and the second glycated albumin antibody;
washing the antigen-antibody complex of the glycated albumin while it is captured in the magnetic field;
reacting the substrate with the antigen-antibody complex to produce hydrogen peroxide;
measuring the hydrogen peroxide; and
obtaining the ratio between the amount of the glycated albumin and the amount of total albumin from the measured amount of the hydrogen peroxide and the measured total amount of the albumin.

21. The methods of Claim 20, wherein said measuring the total amount of albumin including the glycated albumin and the non-glycated albumin includes optically measuring the albumin.

22. The method of Claim 21, wherein said optically measuring the albumin includes binding an absorbance labeling reagent to the albumin.

23. The method of Claim 22, wherein the absorbance labeling reagent is bromocresol green or bromocresol purple.

24. The method of Claim 22 or 23, wherein said optically measuring the albumin includes measuring the absorbance of the absorbance labeling reagent bound to the albumin.

25. The method of any one of Claims 20 to 24, wherein said optically measuring the albumin is performed prior to allowing the first glycated albumin antibody to recognize the glycated albumin and allowing the second glycated albumin antibody to recognize the glycated albumin.

26. A method for measuring glycated albumin, comprising:
providing a solution including an albumin;
providing a first albumin antibody that specifically recognizes the albumin and is bound to a magnetic carrier;
providing a second albumin antibody that specifically recognizes the albumin and is modified with a second oxidase;
providing a second substrate which reacts with the second oxidase;
allowing the first albumin antibody to recognize the albumin;
allowing the second albumin antibody to recognize the albumin;
using a magnetic field to capture an antigen-antibody complex of the albumin recognized by the first albumin antibody and the second albumin antibody in the magnetic field;
washing the albumin antigen-antibody complex while it is captured in the magnetic field;
reacting the second substrate with the antigen-antibody complex of the albumin recognized by the first albumin antibody and the second albumin antibody to produce second hydrogen peroxide;
measuring the second hydrogen peroxide;
providing a first glycated albumin antibody that specifically recognizes the glycated albumin included in the albumin and is bound to a magnetic carrier;
providing a second glycated albumin antibody that specifically recognizes the glycated albumin and is modified with a first oxidase;
providing a first substrate which reacts with the first oxidase;
allowing the first glycated albumin antibody to recognize the glycated albumin;
allowing the second glycated albumin antibody to recognize the second glycated albumin;
using a magnetic field to capture an antigen-antibody complex of the glycated albumin recognized by the first glycated albumin antibody and the second glycated albumin antibody in the magnetic field;
washing the glycated albumin antigen-antibody complex while it is captured in the magnetic field;
reacting the first substrate with the antigen-antibody complex of the glycated albumin recognized by the first glycated albumin antibody and the second glycated albumin antibody to produce first hydrogen peroxide;
measuring the first hydrogen peroxide; and
obtaining the ratio between the amount of the glycated albumin and the amount of total albumin from the measured amount of the first hydrogen peroxide and the measured amount of the second hydrogen peroxide.

27. An apparatus for measuring an antigen, comprising:
an inlet for introducing a solution including an antigen;
a first antibody capable of specifically binding to the antigen and bound with a magnetic particle;
a second antibody capable of specifically binding to the antigen and bound to an oxidase;
a reaction tank for containing an antigen-antibody complex obtained by reacting the first antibody and the second antibody with the antigen;
a magnetic field generator configured to generate a magnetic field in the reaction tank;
a substrate liquid tank containing a substrate liquid including a substrate which reacts with the oxidase, and being fluidly connected in an openable and closable manner with the reaction tank; and
a hydrogen peroxide sensor for measuring hydrogen peroxide generated from the reaction of the oxidase and the substrate.

28. The measuring apparatus of Claim 27, wherein the reaction tank contains a first antibody which is capable of specifically binding to the glycated protein and bound with a magnetic particle, and a second antibody which is capable of specifically binding to the glycated protein and bound with a glucose oxidase.

29. The measuring apparatus of Claim 28, further comprising a buffer tank containing a buffer and fluidly connected to the reaction tank in an openable and closable manner.

30. The measuring apparatus of Claim 29, wherein the buffer tank is configured to separate contaminants from the antigen-antibody complex by introducing the buffer from the buffer tank into the reaction tank after the antigen-antibody complex is formed.

31. The measuring apparatus of Claim 29 or 30, further comprising a hydrogen peroxide measurement tank containing the hydrogen peroxide electrode and fluidly connected in an openable and closable manner to the reaction tank.

32. The measuring apparatus of Claim 31, wherein the buffer tank is configured to send the generated hydrogen peroxide to the hydrogen peroxide measurement tank by introducing the buffer from the buffer tank into the reaction tank, after the hydrogen peroxide is generated.

33. The measuring apparatus of Claim 32, further comprising a drain tank configured to contain a solution discharged from the hydrogen peroxide measurement tank.

34. The measuring apparatus of any one of Claims 27 to 33, further comprising a liquid feeding mechanism for feeding the solution in the apparatus.

35. The measuring apparatus of Claim 29 or 30, wherein the hydrogen peroxide sensor is disposed in the reaction tank.

36. The measuring apparatus of Claim 35, further comprising a drain tank configured to contain a solution discharged from the reaction tank.

37. The measuring apparatus of any one of Claims 27 to 36, wherein the hydrogen peroxide sensor includes a hydrogen peroxide electrode.
